(19) **Europäisches Patentamt**
**European Patent Office**
**Office européen des brevets**

(11) **EP 3 126 838 B1**

(12) # EUROPEAN PATENT SPECIFICATION

(45) Date of publication and mention
of the grant of the patent:
**01.11.2017 Bulletin 2017/44**

(51) Int Cl.:
*G01N 33/532* *(2006.01)*      *G01N 33/535* *(2006.01)*
*G01N 33/74* *(2006.01)*       *G01N 33/543* *(2006.01)*
*C07D 209/16* *(2006.01)*

(21) Application number: **15741886.4**

(22) Date of filing: **01.04.2015**

(86) International application number:
**PCT/EP2015/057138**

(87) International publication number:
**WO 2015/150436 (08.10.2015 Gazette 2015/40)**

(54) **HIGH SENSITIVE DETECTION OF MELATONIN**

HOCHEMPFINDLICHER NACHWEIS VON MELATONIN

DÉTECTION À SENSIBILITÉ ÉLEVÉE DE MÉLATONINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.04.2014 EP 14163378**

(43) Date of publication of application:
**08.02.2017 Bulletin 2017/06**

(73) Proprietor: **Koninklijke Philips N.V.
5656 AE Eindhoven (NL)**

(72) Inventor: **VAN ROOSMALEN, Markus Hendrikus
NL-5656 AE Eindhoven (NL)**

(74) Representative: **Cohen, Julius Simon
Philips Intellectual Property & Standards
High Tech Campus 5
5656 AE Eindhoven (NL)**

(56) References cited:
**WO-A1-2013/063086**

- **MANZ B ET AL: "Development and validation of
a radioimmunoassay for serum melatonin.",
JOURNAL OF CLINICAL CHEMISTRY AND
CLINICAL BIOCHEMISTRY. ZEITSCHRIFT FÜR
KLINISCHE CHEMIE UND KLINISCHE
BIOCHEMIE OCT 1989, vol. 27, no. 10, October
1989 (1989-10), pages 797-802, XP055137427,
ISSN: 0340-076X**

- **TIEFENAUER L X ET AL: "Prevention of bridge
binding effects in haptenic immunoassay
systems exemplified by an iodinated
radioimmunoassay for melatonin", JOURNAL OF
IMMUNOLOGICAL METHODS, ELSEVIER
SCIENCE PUBLISHERS B.V.,AMSTERDAM, NL,
vol. 74, no. 2, 30 November 1984 (1984-11-30),
pages 293-298, XP023974507, ISSN: 0022-1759,
DOI: 10.1016/0022-1759(84)90296-5 [retrieved on
1984-11-30]**

- **IAN D TOMLINSON ET AL: "Biotin tethered
homotryptamine derivatives: High affinity probes
of the human serotonin transporter (hSERT)",
BIOORGANIC & MEDICINAL CHEMISTRY
LETTERS, PERGAMON, AMSTERDAM, NL, vol.
21, no. 6, 21 January 2011 (2011-01-21), pages
1678-1682, XP028169553, ISSN: 0960-894X, DOI:
10.1016/J.BMCL.2011.01.102 [retrieved on
2011-02-02]**

- **Anonymous: "Instructions for Use: Enzyme
immunoassay for the in-vitro-diagnostic
quantitative determination of melatonin in human
serum and plasma. RE54021", , 20 January 2012
(2012-01-20), XP055137612, Retrieved from the
Internet:
URL:http://www.ibl-japan.co.jp/datasheet/8
2086_j.pdf [retrieved on 2014-09-02]**

- **SCHUMACHER M ET AL: "Advanced
immunoassays for the direct determination of
melatonin in human serum and culture media.",
ADVANCES IN EXPERIMENTAL MEDICINE AND
BIOLOGY 1999, vol. 460, 1999, pages 467-472,
XP009179967, ISSN: 0065-2598**

**Description**

FIELD OF THE INVENTION

The present invention relates to the use of a derivative of melatonin in an assay, wherein said derivative is a conjugate at position 3 of melatonin's indole ring and wherein said conjugate comprises a linker of at least 2 carbon atoms, with the proviso that the conjugate does not comprise a polypeptide or protein antigen. The derivative preferably comprises 3-(2-ethylamidoglutaric acid)-5-methoxyindole (GUS) and is coupled to a carrier such as dextran. The invention further relates to a method for detecting and/or quantifying melatonin in a sample using a compound comprising said derivative of melatonin, a corresponding immunobiological assay and a kit of parts for detecting and/or quantifying melatonin based on the melatonin derivative.

BACKGROUND OF THE INVENTION

Melatonin (N-acetyl-5-methoxytryptamine) is a hormone of the pineal gland and involved in many physiological functions, e.g. the regulation of circadian rhythms and seasonal reproduction. Melatonin has been associated with several disorders or physiological problems including depression, sleep disturbances, migraine attacks, regulation of the immune system or human reproduction. In particular, the human circadian rhythm (i.e. the 24-hour biological clock) is highly regulated and dependent in the daily light-dark cycle. Melatonin produced during the night phase of the circadian rhythm can be used to establish suspected problems in the patient's circadian rhythm.

Detection of melatonin in humans is mostly performed on specific sample types such as saliva or extracted plasma samples using immunological or HLPC detection technologies (Voultsios et al., 1997, Journal of Biological Rhythms, 12: 457 -466; Römsing et al., 2006, Scand. J. Clin. Lab. Invest, 66: 181-190). The immunological detection of melatonin typically relies on specific antibodies reactive towards melatonin, which are incubated together with melatonin conjugates or melatonin radioactive labels to determine the amount of captured melatonin from samples. Since melatonin is too small to be capable of producing antisera on its own, it is typically coupled to an antigenic protein such as BSA. Melatonin accordingly works as a hapten with resulting antisera binding to the hapten and adjacent protein structures (Grota et al., 1983, Can J Biochem Cell Biol, 61: 1096-1101).

The first immunological assays used radioactive labels that were later exchanged for enzyme or fluorescent probes (Voultsios et al., 1997, Journal of Biological Rhythms 12: 457 -466). The most widely used conjugates in immunological assays are melatonin derivatives modified such as Melatonin-1-propionic acid, which is modified at the nitrogen of the melatonin molecule and ensures specificity of the assay in combination with the antibody used. Normally, sensitive (<10pg/ml) immunological detection of melatonin relies on long incubation times (3h-16h) combined with low assay temperatures of about 4 to 20°C (Chegini et al., 1995, Clin. Chem, 41: 381-386; or Di et al., 1998, Clin Chem, 44: 304-310).

There is hence a need for an effective technique which allows for a highly sensitive detection of melatonin at temperatures of about 20°C and above and during shorter incubation times.

OBJECTS AND SUMMARY OF THE INVENTION

The present invention addresses these needs and provides means and methods for a highly sensitive detection of melatonin at temperatures of about 20°C and above and during incubation times of less than 16 h, in particular of only about 0.5 h. The above objective is in particular accomplished by a use of a derivative of melatonin in an assay, wherein said derivative is a conjugate at position 3 of melatonin's indole ring and wherein said conjugate comprises a linker of at least 2 carbon atoms, with the proviso that the conjugate does not comprise a polypeptide or protein antigen. The inventors provide the surprising solution that upon the conjugation of melatonin at its 3 position (i.e. at position 3 of the indole ring of melatonin) with a linker of a length of at least 2 C-atoms melatonin derivatives are obtained which, when used in an immunobiological assay or method, allow for a performance of a melatonin detection or quantification assay at 20°C and above for a drastically shortened incubation time of less than 16 h, e.g. for only 30 minutes. This principle has in particular been proven for the melatonin analog 3-(2-ethylamidoglutaric acid)-5-methoxyindole (GUS) which was further bound to dextran.

Thus, in a preferred embodiment of the present invention, said derivative of melatonin is or comprises 3-(2-ethylamidoglutaric acid)-5-methoxyindole (GUS).

In a further preferred embodiment said derivative is coupled to a carrier.

Particularly preferred is dextran as carrier, while other carriers may also be used.

In yet another preferred embodiment, said carrier may further be coupled to a particle. In yet another preferred embodiment, the carrier coupled to the melatonin derivative as defined above may be bound to a surface. In specific embodiments, said carrier may be coating a surface.

In another preferred embodiment said compound comprising a melatonin derivative and a carrier is labeled.

In a further preferred embodiment said assay is an immunobiological melatonin assay with a sensitivity of <10 pg melatonin/ml. In a particularly preferred embodiment, said assay is an immunobiological melatonin quantification assay with a sensitivity of <10 pg melatonin/ml.

In yet another preferred embodiment, said assay is performed at a temperature of more than about 20°C. In a particularly preferred embodiment, said assay is performed at a temperature of about 20°C to 25°C. In another preferred embodiment, said assay is performed for less than 16 h. In a particularly preferred embodiment, said assay is performed for about 0.5 h

In another aspect the present invention relates to a method or immunobiological assay for detecting and/or quantifying melatonin in a sample, comprising the steps of:

(a) incubating a sample with a predefined amount of a compound comprising a derivative of melatonin as mentioned herein above;
(b) binding of melatonin and/or the compound comprising said melatonin derivative with a melatonin binding molecule, wherein said melatonin binding molecule is immobilized on a surface;
(c) measuring the amount of the compound comprising said melatonin derivative bound to said melatonin binding molecule; and
(d) detecting the presence of melatonin in the sample and/or deducing the amount of melatonin in the sample based on the inverse proportion of the measured amount of the compound comprising said melatonin derivative.

In another aspect the present invention relates to a method or immunobiological assay for detecting and/or quantifying melatonin in a sample, comprising the steps of

(a) providing a compound comprising a derivative of melatonin as mentioned herein above in an immobilized form on a surface, or in an immobilizable form;
(b) incubating a sample on said surface with a predefined amount of a melatonin binding molecule thereby allowing for the binding of the melatonin binding molecule to melatonin and/or the immobilized or immobilizable compound comprising said melatonin derivative;
(c) measuring the amount of said melatonin binding molecule bound to the immobilized compound comprising said melatonin derivative; and
(d) deducing the amount of melatonin in the sample based on the inverse proportion of the measured amount of the melatonin binding molecule.

In a preferred embodiment of said method or assay said binding molecule is a melatonin specific antibody.

In another preferred embodiment of said method or assay, said method or assay has a sensitivity of <10 pg melatonin/ml, performed at a temperature of more than about 20°C and/or performed for less than 16 h. In a particularly preferred embodiment, said method or assay is performed at a temperature of about 20°C to 25°C.

In further particularly preferred embodiments, said method or assay is performed for about 0.5 h.

In a further preferred embodiment, said method or assay is performed in a device allowing magnetic actuation of particles.

It is further preferred that said sample as mentioned above is a saliva, blood, serum, plasma, urine, or sweat sample.

In a final aspect the invention relates to a kit of parts for detecting and/or quantifying melatonin, comprising a derivative of melatonin as mentioned herein above and a melatonin specific antibody.

BRIEF DESCRIPTION OF THE DRAWINGS

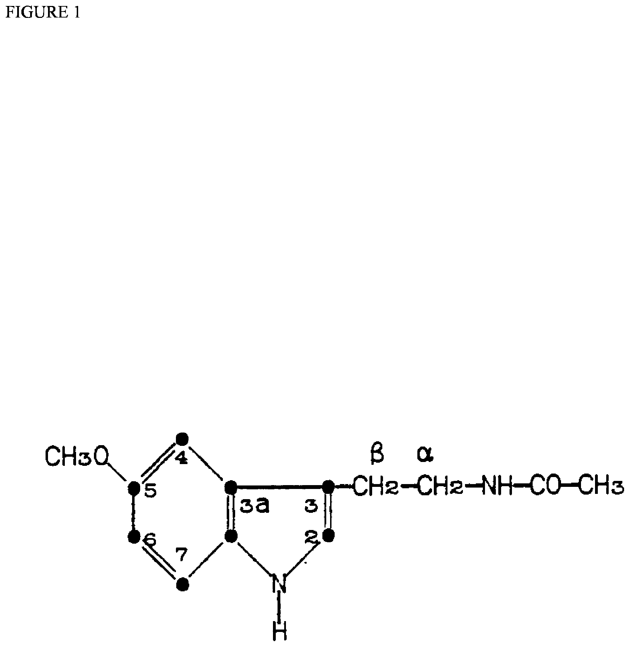

Fig. 1 shows the chemical structure of melatonin include its indole ring with the relevant positions indicated.

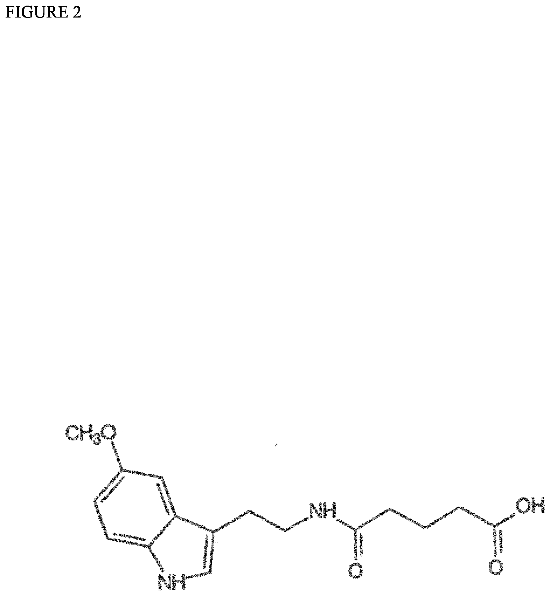

Fig. 2 depicts melatonin derivative GUS 3-(2-ethylamidoglutaric acid)-methoxyindole), which is derivative of melatonin at position 3.

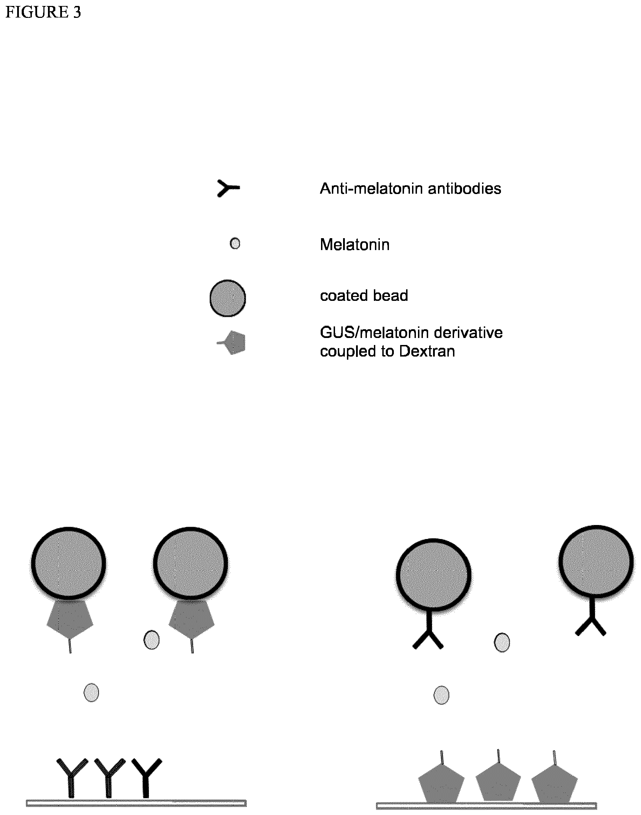

Fig. 3 exemplifies in the left-hand panel an assay format with a melatonin derivative comprising a carrier that is coupled to beads (particles) which are detected by binding molecules (antibodies) coated/printed on the surface, in case of absence of melatonin. Presence of melatonin in samples will prevent the formation of the complex and results in reduced signals. In the right-hand panel a further exemplary embodiment of an assay format according to the present invention is illustrated, in which a melatonin derivative comprising a carrier is immobilized on the surface, while antibodies which are coupled to beads (particles) can bind to said melatonin derivative in case of absence of melatonin. Presence of melatonin in samples will prevent the formation of the complex and results in reduced signals.

Fig. 4 shows the results of an comparison of assay formats with the reference conjugate and GUS conjugate at an assay temperature of 20°C and 30°C (for the GUS conjugate) and melatonin dilution series in plasma. The X indicates the values at 30°C, the full circle indicates the values at 20°C.

Fig. 5 shows the temperature dependence of tested conjugates. Indicated in black is the measured reference, indicated in white is the measured GUS-comprising compound.

DETAILED DESCRIPTION OF THE EMBODIMENTS

The present invention relates to the use of a derivative of melatonin in an assay.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20 \%$, preferably $\pm 15 \%$, more preferably $\pm 10 \%$, and even more preferably $\pm 5 \%$.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The present invention concerns in one aspect a use of a derivative of melatonin in a detection procedure for melatonin, in particular an assay, wherein said derivative is a conjugate at position 3 of melatonin's indole ring and wherein said conjugate comprises a linker of at least 2 carbon atoms, with the proviso that the conjugate does not comprise a polypeptide or protein antigen.

The term "derivative of melatonin" as used herein refers to a chemically modified version of the melatonin molecule, which maintains its core structure and function as immunological hapten. In a preferred embodiment, a derivative of melatonin according to the present invention is detectable with a high affinity with an antibody raised against melatonin, e.g. a melatonin- bovine serum albumin (BSA) conjugate, a melatonin-thyroglobuline (TG) conjugate or a melatonin-ovalbumin (OVA) conjugate.

The term "conjugate at position 3 of melatonin's indole ring" as used herein means that the melatonin structure is chemically modified at the position 3 of the core indole ring of melatonin. The conjugation process may, in certain embodiments, result in an extension of melatonin's side chain $-CH_2-CH_2-NH-CO-CH_3$ at its terminal methyl group, or, in other embodiments, in a replacement or modification of one or more other non-terminal groups of said side chain. For the conjugation one or more suitable conjugation reagents can be used. A conjugation reagent can, for instance, include one or more conjugation moieties, e.g., chemical moieties suitable for conjugating to a moiety on a secondary compound. In some cases, a conjugation reagent can be a monovalent aldehyde (e.g., formaldehyde), bivalent aldehyde (e.g., glutaric aldehyde), hydrozine, carbodiimide, isocyanate or diisocyanate. Typically, conjugation reagents are molecules that contain two or more reactive ends capable or chemically attaching to specific functional groups on melatonin or other molecules such as primary amine ($-NH2$), carboxyls ($-COOH$), sulflrydryls, or carbonyls ($-CHO$), or reactive groups such as carbodiimide (e.g., EDC), NHS ester, imidoester, PFP ester, hydroxymethyl phosphine, maleimide, haloacetyl (bromo- or iodo-), pyridyldisulfide, vinyl sulfone, hydrazide, diazirine, or aryl azide.

In preferred embodiments, the conjugate at position 3 of melatonin's indole ring comprises a linker molecule of at least 2 carbon atoms. In case of an extension of melatonin's side chain $-CH_2-CH_2-NH-CO-CH_3$ at its terminal methyl group, said linker is provided in extension to said side chain. In case of a replacement or modification of one or more other non-terminal groups of said side chain, said linker is provided at the site of modification. The linker may be a branched molecule or a nonbranched molecule. The linker may, for example, be a hydrocarbon molecule of different composition and/or length. The length of the molecule may vary between about 2 C atoms to about 50 C atoms, or more. Also envisaged are other lengths or any length value within the indicated range. The hydrocarbon molecule may, for example, have a length of 2, 3, 4, 5, 6, 7, 8, 9, 10, 15, 20, 25, 30, 35, 40, 45, or 50 C atoms or any other number of C atoms between the indicated values. In addition to C atoms, a linker may further comprise other atoms or functionalities, e.g. N, S, O, or P-atoms or corresponding groups, including side chains etc.

The linker may, in alternative embodiments, be or comprise or consist of a carbohydrate, i.e. an oligo- or polysaccharide molecule. The carbohydrate molecule may, for example, have a length of 2, 3, 4, 5, 6, 7, 8, 9, 10, 15, 20, 25, 30, 35, 40, 45, or 50 C atoms or any other number of C atoms between the indicated values. The carbohydrate may be based on monomeric sugars such as glucose, glucopyranose, galactose, mannose, ribose etc., or on oligosaccharide units such as maltose, sucrose or lactose etc., or derivatives thereof. The carbohydrate may further comprise polymeric sugars such as glucan, mannan, galactan, cellulose, chitin, pectin or dextran.

The linker may, in alternative embodiments, be or comprise or consist of a lipid. Lipids constitute a broad group of naturally occurring molecules including, but not limited to, fats, waxes, styrols, fat-soluble vitamins (such as vitamins A, D, E and K), monoglycerides, digycerides, phospholipids. Such lipids are typically built of a lipid tail and a headgroup. Examples of suitable lipids include phospholipids, e.g. phosphatidyl ethanolamine, phosphatidylcholine, egg phosphatidyl-ethanolamine, dioleoylphosphatidyl ethanolamine. Particularly preferred are the phospholipids MPPC and DPPC. The lipids may be of different compositions and/or lengths. The length of the molecule may vary between 2 C atoms to about 100 C atoms, or more. Also envisaged are other lengths or any length value within the indicated range. The lipid molecule may, for example, have a length of 2, 3, 4, 5, 6, 7, 8, 9, 10, 15, 20, 25, 30, 35, 40, 45 or 50 C atoms or any other number of C atoms between the indicated values.

The linker may, in further alternative embodiments, be or comprise or consist of a non-biological polymer. The term "non-biological polymer" as used herein refers to a polymer which is not from a biological source (biopolymer) and is chemically synthesized. These polymers may preferably also comprise a number up to about 50 atoms. Suitable polymers for this purpose have been described in the art (e.g. by Ratner, B.D.; Hoffman, A.S.; Schoen, F.J.; Lemons, J.E., Biomaterials Science, 2nd Ed.; Eds.; Elsevier: London, 2004). Examples of a suitable non-biological polymers are polymers such as poly(glycolic acid) (PGA) or poly(lactic acid) (PLA), poly(caprolactone) (PCL, poly(*N*-vinyl-2-pyrrolidone) (PVP), *polydioxanone (PDS),* or poly(ethylene glycol) (PEG) or copolymers thereof also termed hetereopolymers that are derived from two (or more) monomeric species. Further envisaged are block copolymers with two or three distinct blocks which are called diblock copolymers and triblock copolymers, respectively. Preferred block copolymers include, but are not limited to p*oly(lactide-co-glycolide) copolymers (PLGA),* poly(ethylene oxide)-poly(propylene oxide) (PEP-PPO) poly(ethylene oxide)-poly(propylene oxide) poly(ethylene oxide) (PEP-PPO-PEO), poly(ethylene oxide)-block-poly(*L-lactide)* *(PEG-PLLA),* poly(ethylene oxide)-block-poly(caprolactone) (PEG-PCL), poly(ethylene glycol)-*block*-poly($\alpha$-hydroxy acid) (PEG-PHA), PAMAM (Gen 4-7) or Pluronic P-105. It is preferred that the polymer has a non-protein like or non-polypeptide like structure and/or form.

In another group of specific embodiments, the linker may be or comprise or consist of polyethylene glycol molecules. Polyethylene glycols envisaged by the present invention may be of different compositions and/or lengths. Preferred polyethylene glycol (PEG) variants include polydisperse or monodisperse PEG molecules. Particularly preferred is monodisperse PEG. PEGs may further be branched. The PEGs present in the derivative may preferably comprise a number up to about 50 atoms.

In further embodiments one or more of the above mentioned linkers may be combined, e.g. a PEG may be combined with one or more lipid linkers, or a carbohydrate linker may be combined with one or more PEG linkers etc.

The present invention excludes that the conjugate as defined above is a, or comprises a polypeptide or protein antigen. The invention thus envisages a derivative of melatonin with the proviso that said derivative does not comprise or is linked to a polypeptide antigen or protein antigen. In particular, the invention thus envisages a derivative of melatonin with the proviso that said derivative does not comprise or is linked to a polypeptide antigen or protein antigen such as bovine serum albumin (BSA), cationized BSA, thyroglobuline from porcine (TG), keyhole limpet hemocyanin (KLH), ovalbumin (OVA), tetanus toxoid, or gelatin. Such antigenic proteins are typically used in the field for conjugation to small haptens in order to raise suitable antibodies against the hapten and parts of the antigen. As the present inventors surprisingly found, such polypeptide/protein conjugates are not necessary for performing immunological melatonin assays in view of conjugations to melatonin at position 3 of its indole ring as defined herein above and below. The performance of immunological melatonin assays can hence be improved by using conjugations to melatonin at position 3 of its indole ring as defined herein above and below, although antibodies or binding molecules employed in such assays may have been obtained with classical melatonin-polypeptide/protein conjugates.

In specific embodiments a melatonin derivative according to the present invention does not comprise or is linked to a polypeptide antigen or protein antigen such as bovine serum albumin (BSA), cationized BSA, thyroglobuline (TG), keyhole limpet hemocyanin (KLH), ovalbumin (OVA), tetanus toxoid, or gelatin in the vicinity of melatonin's core structure, e.g. its indole ring, such that the polypeptide or protein antigen, together with the melatonin derivative as an hapten, provides an immunological active or immunologically recognizable entity, e.g. recognizable by monoclonal or polyclonal antibodies.

In a particularly preferred embodiment, the melatonin derivative is or comprises 3-(2-ethylamidoglutaric acid)-5-methoxyindole (GUS). In further envisaged embodiments the melatonin derivative is 3-(2-ethylamidohexanedioic acid)-5-methoxyindole, 3-(2-ethylamidobutanoic acid)-5-methoxyindole, 3-(2-ethylamidopentanoic acid)-5-methoxyindole, 3-(2-ethylamidoheptanedioic acid)-5-methoxyindole, or 3-(2-ethylamidooctanedioic acid)-5-methoxyindole.

A melatonin derivative as defined herein above, e.g. GUS, may further be coupled to a carrier entity. The term "carrier entity" as used herein relates to a non-proteinous entity which allows to stably bind and/or present the melatonin derivative. Such carrier entity may be a polymeric or monomeric molecule comprising more than about 40 to 50 atoms. For example, the carrier may be a hydrocarbon with a length of about 40 to 500 C atoms, or more. Also envisaged are other lengths or any length value within the indicated range. The hydrocarbon molecule may, for example, have a length of 40, 45, 50, 55, 60, 65, 70, 75, 80, 85, 90, 95, 100, 200, 300, 400 or 500 C atoms or any other number of C atoms between the indicated values. In addition to C atoms, a linker may further comprise other atoms or functionalities, e.g. N, S, O, or P-atoms or corresponding groups, including side chains etc. The present invention envisages that one melatonin derivative as defined herein, e.g. one molecule of GUS, be linked to one carrier entity, or that more than one melatonin derivative as defined herein, e.g. several molecules of GUS, be linked to one carrier entity. For example, there may be 1, 2, 3, 4, 5, 6, 7, 8, 9, 10, 11, 12, 13, 14, 15, 20, 25, 30, 35, or 40 molecules, or any number of molecules in between these values, or more than 40 molecules of melatonin derivatives, e.g. GUS, be coupled to one carrier entity. It is preferred that the number of melatonin derivatives per carrier entity is between 2 and 10, or between 3 and 8. Particularly preferred is a number of melatonin derivatives per carrier entity of 5.

The carrier may, in alternative embodiments, be or comprise or consist of a carbohydrate, i.e. a polysaccharide molecule. The carbohydrate molecule may, for example, have a length of 40, 45, 50, 55, 60, 65, 70, 75, 80, 85, 90, 95, 100, 200, 300, 400 or 500 C atoms or any other number of C atoms between the indicated values. In alternative embodiments, the carbohydrate molecule may have 10 to 100 sugar ring structures, e.g. 10, 20, 30, 40, 50, 60, 70, 80, 90, 100, or more than 100 sugar ring structures, or any value in between these values. The carbohydrate may be or comprise polymeric sugars such as glucan, mannan, galactan, cellulose, chitin, pectin or dextran. Particularly preferred is dextran.

The term "dextran" as used herein refers to a complex, branched glucan composed of chains of varying lengths, which may have weights of ranging from 3 to 2000 kilodaltons. The straight chain typically consists of alpha-1,6 glycosidic linkages between glucose molecules, while branches begin from alpha-1,3 linkages. Dextran may be synthesized from sucrose, e.g. by lactic-acid bacteria. In the context of the present invention dextran to be used as carrier may preferably have a molecular weight of about 15 to 1500 kilodalton. It is particularly preferred that the dextran has a molecular weight of about 40 to 500 kilodalton, e.g. 40, 50, 60, 70, 80, 90, 100, 120, 140, 150, 160, 180, 200, 220, 240, 250, 260, 280, 300, 320, 340, 350, 360, 380, 400, 420, 440, 450, 460, 480, or 500 kilodalton, or any value in between these values.

Another example of a suitable carrier is a lipid such as a phospholipid, e.g. phosphatidyl ethanolamine, phosphatidylcholine, egg phosphatidyl-ethanolamine, dioleoylphosphatidyl ethanolamine with a length of about 40 C atoms to about 500 C atoms, or more. Also envisaged are other lengths or any length value within the indicated range. The lipid molecule may, for example, have a length of 40, 45, 50, 55, 60, 65, 70, 75, 80, 85, 90, 95, 100, 200, 300, 400 or 500 C atoms or any other number of C atoms between the indicated values.

Further examples of carrier molecules include polyethylene glycol (PEG) molecules as mentioned above. The carrier PEGs may have about 40 C atoms to 500 C atoms or more. The PEGs may be polydisperse or monodisperse PEG molecules, or be branched, e.g. having 3-10 PGE chains emanating from a central core group, be star PEGs having 10 to 100 PEG chains emanating from a central core group, or be comb PEGs which have multiple PEG chains grafted to a different polymer or linear PEG backbone. According to the PEG's average molecular weights, envisaged PEGs may be PEG 10,000, PEG 12,000, PEG 15,000, PEG 20,000 or PEGs having higher molecular weights. Particularly preferred is a PEG larger than PEG 10,000. Smaller PEG molecules such as, for example, PEG 400, PEG 600, PEG 800, PEG 1000, PEG 1500, PEG 2000, PEG 3350, PEG 4000, PEG 6000, or PEG 8000 are also envisaged.

The carrier may, in further alternative embodiments, also be or comprise or consist of a non-biological polymer such as poly(glycolic acid) (PGA) or poly(lactic acid) (PLA), poly(caprolactone) (PCL, poly(*N*-vinyl-2-pyrrolidone) (PVP), *polydioxanone (PDS),* or poly(ethylene glycol) (PEG), poly*(lactide-co-glycolide) copolymers (PLGA),* poly(ethylene oxide)-poly(propylene oxide) (PEP-PPO) poly(ethylene oxide)-poly(propylene oxide) poly(ethylene oxide) (PEP-PPO-PEO), poly(ethylene oxide)-block-p*oly(L-lactide) (PEG-PLLA),* poly(ethylene oxide)-block-poly(caprolactone) (PEG-PCL), poly(ethylene glycol)-*block*-poly($\alpha$-hydroxy acid) (PEG-PHA), PAMAM (Gen 4-7) or Pluronic P-105.

A carrier as defined herein above, which is coupled to a melatonin derivative according to the present invention, may further be coupled to a particle. Alternatively, such a carrier may be bound to a surface. If bound to a surface, for example a coated surface may be provided.

The coupling to a particle or surface may either be a single coupling such that a single melatonin derivative or a single compound comprising a melatonin derivative and a carrier as defined herein is bound to one particle or a surface, or the coupling may involve several or many melatonin derivatives or compounds comprising a melatonin derivative and a carrier per particle or per surface. For example, there may be about 10, 15, 20, 25, 30, 40, 50, 60, 70, 80, 90, 100, 200, 300, 400, 500 or more than 500 or any number between these values of melatonin derivatives or compounds comprising a melatonin derivative and a carrier per particle or per surface. For the coating of surfaces it is preferred having a suitably dense arrangement of melatonin derivatives or compounds comprising a melatonin derivative of about 2000 to 10.000 elements per particle or per defined area. It is particularly preferred that about 1 to 100 μg/mg dextran is coupled to a particular or surface area, e.g. 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95 or 100 μg/mg. Even more preferred is the coupling of about 10 μg/mg dextran. In further preferred embodiments, this amount of dextran molecules may be coupled to a number of melatonin derivatives per carrier entity as define herein above, preferably to about 1 to 10 such melatonin derivatives, e.g. to 2, 3, 4, 5, 6, 7, 8, 9 or 10 melatonin derivatives, e.g. GUS.

The term "particle" as used herein means a small, localized object to which can be ascribed a physical property such as volume or mass. In the context of the present invention a particle comprises or consists of any suitable material known to the person skilled in the art, e.g. the particle may comprise, or consist of, or essentially consist of inorganic or organic material. Typically, a particle may comprise, or consist of, or essentially consist of metal or an alloy of metals, or an organic material, or comprise, or consist of, or essentially consist of carbohydrate elements. Examples of envisaged material include agarose, polystyrene, latex, polyvinyl alcohol, silica and ferromagnetic metals, alloys or composition materials. Particularly preferred are magnetic or ferromagnetic metals, alloys or compositions. Particularly preferred particles useful in the present invention are superparamagnetic particles. The term "superparamagnetic" as used herein describes a form of magnetism, which appears in small ferromagnetic or ferromagnetic nanoparticles. It is known in the art that in sufficiently small nanoparticles, magnetization can randomly flip direction under the influence of temperature. The time between two flips is referred to as the Néel relaxation time. In the absence of an external magnetic field, when the time used to measure the magnetization of the nanoparticles is much longer than the Néel relaxation time, the magnetization appears to be in average zero, i.e. in the paramagnetic state. In such a state an external magnetic field is able to magnetize the nanoparticles similarly to a paramagnet. However, the magnetic susceptibility is much larger than those of paramagnets. In further preferred embodiments, the material may have specific properties. The material may, for example, be magnetic or be non-magnetic. The material may, in other embodiments, be hydrophobic, or hydrophilic. In further specific embodiments the particle is a plastic particle. Examples of plastic particles include latex or polystyrene beads, e.g. those commonly used for purification. In yet another embodiment, the particle may be a cell like particle. The term "cell like particle" as used herein refers to a biological or semi-biological structure, which is present in biological systems or has the form and/or function of biological systems or parts of biological systems. In further specific embodiments, a particle may comprise, essentially consist of or consist of sepharose or agarose. The particle may, in further embodiments, be or comprise round shaped support or substrate structures. The particles may, in another group of embodiments, be organized in arrays or geometrical forms, e.g. located at predefined points or spots, or may be provided in an irregular manner. Particles may further overlay a substrate ground or be fixed on substrate ground by connector elements such as rods etc. Particles may further be coated with protective coatings, e.g. PEG molecules, and/or with functionalized coatings, e.g. comprising biochemically active compounds. Envisaged particles include magnetic particles with suitable functional groups such as epoxy groups. Further envisaged examples include carboxyl (COOH) microparticles. Such microparticles may be used for covalent coupling of molecules by activating the carboxyl groups with water-soluble carbodiimide. The carbodiimide may further react with the carboxyl group allowing the creation of an active ester that may be reactive toward primary amines.

Furthermore, a particle essentially behaves as a whole unit in terms of its transport and properties. Particles may accordingly be of a symmetrical, globular, essentially globular or spherical shape, or be of an irregular, asymmetric shape or form.

The size of a particle envisaged by the present invention typically ranges between 50 nm and 50 μm. Preferred are particles in the nanometer and micrometer range up to several micrometers. In further preferred embodiments the particle diameter is larger than 100 nm. The term "diameter" as used herein refers to any straight line segment that passes through the center of the particle and whose endpoints are on the particle surface. In case of non-spherical or semi spherical particles, the diameter is understood as the average diameter of the largest and shortest straight line segments that pass thought the center of the particle and whose endpoints are on the particle surface. It is further understood that a radius of a particle as defined herein is half of its diameter as defined herein above. Particularly preferred are nanoparticles, e.g. particles of a diameter of about 100 nm to 10 micrometer, more preferably 100 nm to 3 μm, even more preferably 300 nm to 1000 nm, e.g. 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, 550 nm, 560 nm, 570 nm, 580 nm, 590 nm, 600 nm, 620 nm, 650 nm, 670 nm, 700 nm, 720 nm, 750 nm, 770 nm, 800 nm, 820 nm, 850 nm, 870 nm, 900 nm, 920 nm, 950 nm, 970 nm, 1000 nm, or any value in between. Even more preferred are nanoparticles having a diameter of about 500 nm.

A "surface" according to the present invention can have any structure and may comprise molecules or a network of molecules suitable to cover a surface. A surface may, for example, be the surface of flat sensor surface or, in specific embodiments, a device, cartridge, microfluidic chamber, reaction chamber or particle surface. The envisaged surface may be the site of recognition, binding and/or subsequent detection of a target molecule of interest, e.g. of an antibody or any other suitable binding molecule.

The compound comprising a melatonin derivative and a carrier may be labeled. Such a labeling activity may be based on conjugation reactions using various chemistries, e.g. aldehyde, carboxylic acids, or amine reactions. The label may be any suitable label known to the skilled person, e.g. a radioactive label, an enzymatic label, a fluorescent label, a chemiluminescent label or a bioluminescent label. Examples of labels that can be conjugated to a melatonin derivative according to the invention include fluorescent dyes or metals (e.g. fluorescein, rhodamine, phycoerythrin, fluorescamine), chromophoric dyes (e.g. rhodopsin), chemiluminescent compounds (e.g. luminal, imidazole), fluorescent polypeptides (e.g. green fluorescent protein (GFP)) and bioluminescent proteins (e.g. luciferin, luciferase), haptens (e.g. biotin), and contrast agents such as USPIOS or 19Fluor. Further labels which may be used within the context of the present invention include 6-FAM, HEX, TET, ROX, Cy3, Cy5, Texas Red or Rhodamine, TAMRA, Dabcyl, Black Hole Quencher, BHQ-1 or BHQ-2. Target molecules may also be labeled with radioisotopes e.g. $^{3}$H, $^{14}$C, $^{32}$P, $^{33}$P, $^{35}$S, $^{125}$I, $^{11}$C, $^{13}$N, $^{15}$O, $^{18}$F, $^{64}$Cu, $^{62}$Cu, $^{124}$I, $^{76}$Br, $^{82}$Rb $^{68}$Ga or $^{18}$F. Preferred is the presence or use of a biotin label or of an enzymatic label, e.g. of an enzyme such as horseradish peroxidase, alkaline phosphatase, or beta-lactamase. Particularly preferred is the use or presence of horseradish peroxidase. In a particularly preferred set of embodiments, biotin is employed as label for the compound comprising a melatonin derivative and a carrier. This label may subsequently be bound or allowed to interact with suitable interactors such as streptavidin or similar molecules such as avidin, streptavidin derivatives, avidin-related proteins, avidin-like entities such as tamavidin 1 and 2, bradavidin, or NeutrAvidin etc. The streptavidin (or similar) functionality may be provided with an enzymatic label, e.g. of an enzyme such as horseradish peroxidase, alkaline phosphatase, or beta-lactamase. Particularly preferred is the use of streptavidin-HRP.

The label may be conjugated to a compound comprising a melatonin derivative and a carrier as defined herein above at any suitable position. It is preferred that the label is localized at the carrier portion of the compound. In specific embodiments, the label may be provided at a particle, which is coupled to a compound comprising a melatonin derivative and a carrier. The label may be provided as single label per compound, or as multiple labels, e.g. 2, 3, 4, 5, 6, 7, 8, 9, 10, 15, 20, 30, 40, 50 or more than 50 label molecules per compound. It is preferred that one label be linked to the carrier portion of the compound as defined herein above. The ratio of carrier, compound and label as mentioned above may be any suitable ratio. In a non-limiting example, this ratio may be 1-5 : 5-100 : 1-10, or 1 : 5 : 1, or 1 : 60 : 2. Further ratios are explicitly envisaged by the present invention. In a preferred example, there may be combination of one dextran molecule, coupled to about 5 melatonin derivatives (such as GUS), which is labeled with one biotin entity. In a further example, there may be combination of one dextran molecule, coupled to about 60 melatonin derivatives (such as GUS), which is labeled with 2 biotin entity.

In specific embodiments, the ratio of carrier, melatonin derivative compound and label may be made dependent on the nature and/or molecular weight and/or size and/or length of the carrier. For larger carriers a higher number of melatonin derivative compounds and a higher number of labels is necessary. For example, when using dextran of a molecular weight of 40 kilodalton, a ratio of 1 : 5 : 1 may be used, e.g. one dextran molecule, coupled to about 5 melatonin derivatives (such as GUS), which is labeled with one biotin entity. This ratio may be changed in case of a higher molecular weight of the carrier. For example, in case of a dextran of a molecular weight of 400 kilodalton, a ratio of 1 : 60 : 2 may be used, e.g. one dextran molecule, coupled to about 60 melatonin derivatives (such as GUS), which is labeled with 2 biotin entity. These ratios may be further adapted and changed according to the nature, molecule weight, length or size of the carrier, melatonin derivative compound and/or label. The present invention thus envisages all suitable modifications of the above provided non-limiting ratio examples.

A melatonin derivative as defined herein above, in particular a melatonin derivative coupled to a carrier, or coupled to a carrier and a particle, or coupled to a carrier and a surface as defined herein above may be used for any suitable type of detection procedure. In specific embodiments such melatonin derivative may be used for a detection procedure of melatonin, or of structurally similar molecular. It is preferred that a melatonin derivative coupled to a carrier, or coupled to a carrier and a particle, or coupled to a carrier and a surface as defined herein above may be used in an assay, more preferably in an immunobiological assay. Examples of immunobiological assays in which the melatonin derivative according to the present invention may be used include competitive and non-competitive assay systems using techniques such as western blots, radioimmunoassay like RIA (radio-linked immunoassay), ELISA (enzyme linked immunosorbent assay), "sandwich" immunoassays, immunoradiometric assays, fluorescent immunoassays, e.g. FIA (fluorescence-linked immunoassay), chemiluminescence immunoassays and electrochemiluminescence immunoassay (ECLIA). Details and further features of such assays would be known to the skilled person or can be derived from suitable literature sources such as the ebook Assay Guidance Manual, edited by G. Sitta Sittampalam, Bethesda (MD): Eli Lilly & Company and the National Center for Advancing Translational Sciences; 2004 at http://www.ncbi.nlm.nih.gov/books/NBK53196/, in particular section Immunoassay Methods, Karen L. Cox et al., Eli Lilly & Company, Indianapolis, IN, of 2012, or later updated versions thereof.

Preferred is the use of a melatonin derivative according to the present invention in a competitive immunobiological melatonin assay. Such a competitive assay is in principle base on the incubation of labeled antigen (e.g. a melatonin derivative according to the present invention comprising fluorescent labels or radioactive labels) with a suitable antibody in the presence of increasing amounts of unlabeled antigen (melatonin), and the detection of the antibody bound to the labeled antigen. The competitive assay may in one embodiment be based on melatonin derivatives according to the present invention which are bound to particles and freely floating in a sample or detection liquid, e.g. in a reaction chamber of a device, while suitable antibodies may be immobilized at a surface of such a chamber or device. In an alternative embodiment, the competitive assay may be based on melatonin derivatives according to the present invention which are immobilized on the surface of a reaction chamber of a device, while suitable antibodies, e.g. labeled antibodies, are freely floating in a sample or detection liquid. The number of bound particles on the surface relate thus to the number of unbound melatonin.

The amount of bound melatonin is typically inverse related to the amount of bound conjugate, but relies on the relative affinity of melatonin and the melatonin conjugate to the antibody at a given assay condition. The assay conditions that may influence this ratio can be assay time, temperature, sequence of reactions (melatonin vs conjugate) and the amount conjugate vs antibody. The sensitivity may be determined by measuring the dose that is distinguished from measurements without melatonin, commonly defined as the limit of detection. The present invention also envisages suitable alternatives to this approach, including improvements of the methodology to be developed in the future.

In a particularly preferred embodiment, the assay is an immunobiological melatonin assay with a high sensitivity or limit of detection for melatonin. The sensitivity or limit of detection may be in a range of about 0. 5 to about 10 pg melatonin/ml. For example, the sensitivity may be at 0.5, 1, 2, 3, 4, 5, 6, 7, 8, 9 or 10 pg melatonin/ml, or at any value in between these values, or below. For example, the sensitivity may be <10 pg melatonin/ml, preferably <5 pg melatonin/ml, more preferably <3 pg melatonin/ml, even more preferably <1 pg melatonin/ml.

The assay is in a further preferred embodiment an immunobiological melatonin quantification assay with a high sensitivity for melatonin. The limit of the quantification assay may be in a range of about 0.5 to about 20 pg melatonin/ml. For example, the sensitivity of the quantification assay may be at 0.5, 0.1, 0.5, 1, 2, 3, 4, 5, 6, 7, 8, 9 10, 15 or 20 pg melatonin/ml, or at any value in between these values, or below. For example, the sensitivity of the quantification assay may be <10 pg melatonin/ml, preferably <5 pg melatonin/ml, more preferably <3 pg melatonin/ml, even more preferably <1 pg melatonin/ml.

The melatonin derivative according to the present invention may be used in an assay as defined herein above under any suitable temperature conditions. Such suitable temperature conditions include all temperatures at which the assay can be performed in order to provide reasonable or suitable results, e.g. at which a quantitative detection of melatonin can be performed, or at which a correct statement on the amount of melatonin in a sample can be made. Such temperatures may include low temperatures of about 4°C to high temperatures of about 37°C. It is preferred that the melatonin derivative according to the present invention is used in an assay as defined herein above at a temperature of more than about 18°C, or more than about 20°C. For example, the melatonin derivative according to the present invention may be used at a temperature of 18°C, 19°C, 20°C, 21°C, 22°C, 23°C, 24°C, 25°C, 26°C, 27°C, 28°C, 29°C, 30°C, 31°C, 32°C, 33°C, 34°C or 35°C or any value in between the mentioned values. Preferably, the melatonin derivative according to the present invention is used in an assay as defined herein above at a temperature of 18°C to about 25°C. In the most preferred embodiment, the melatonin derivative according to the present invention is used in an assay as defined herein above at a temperature of about 20°C.

The melatonin derivative according to the present invention may further be used in an assay as defined herein above for a suitable period of time. Such suitable time periods include all periods at which the assay can be performed in order to provide reasonable or suitable results, e.g. at which a quantitative detection of melatonin can be performed, or at which a correct statement on the amount of melatonin in a sample can be made. Such time periods may long assay times of more than 16 hours, or short assay times of less than 16 hours. It is preferred that the assay time is less than 16 hours, e.g. 15 h, 14 h, 13 h, 12 h, 11 h, 10 h, 9 h, 8 h, 7 h, 6 h, 5 h, 4 h, 3 h, 2 h, 1 h, or less than 1 h, or any time period in between the mentioned time periods. In particularly preferred embodiments, the assay time is less than about 1 h, e.g. 50 min, 45 min, 40 min, 35 min, 30 min, 25 min, 20 min, 15 min, 10 min, or 5 min or any time period in between these values. Even more preferably, the assay time is about 30 min.

In another aspect the present invention relates to a method or immunobiological assay for detecting and/or quantifying melatonin in a sample, comprising the steps of: incubating a sample with a predefined amount of a compound comprising a derivative of melatonin as mentioned herein above; binding of melatonin and/or the compound comprising said melatonin derivative with a melatonin binding molecule, wherein said melatonin binding molecule is immobilized on a surface; measuring the amount of the compound comprising said melatonin derivative bound to said melatonin binding molecule; and detecting the presence of melatonin in the sample and/or deducing the amount of melatonin in the sample based on the inverse proportion of the measured amount of the compound comprising said melatonin derivative. The method or immunobiological assay may accordingly be able to at least detect melatonin in a sample. In the alternative, the method immunobiological assay may further be able to quantify the amount of melatonin in a sample. The method or immunobiological assay as mentioned above is in principle based on a competitive binding detection. It may further include additional modification steps.

The detection limit of the method of assay may be in a range of about 0.05 to about 200 pg melatonin/ml. For example, the detection limit of the method or assay may be at 0.5, 1, 2, 3, 4, 5, 6, 7, 8, 9 or 10 pg melatonin/ml, or at any value in between these values, or below. Preferably, the sensitivity of the method or assay is <10 pg melatonin/ml. Similarly, in case of a quantification method or assay, the limit of the quantification method or assay may be in a range of about 0.5 to about 20 pg melatonin/ml. For example, the sensitivity of the quantification assay may be at 0.5, 0.1, 0.5, 1, 2, 3, 4, 5, 6, 7, 8, 9 10, 15 or 20 pg melatonin/ml, or at any value in between these values, or below.

The term "sample" as used herein refers to any biological material obtained via suitable methods known to the person skilled in the art from an individual. The sample used in the context of the present invention should preferably be collected in a clinically acceptable manner, more preferably in a way that analytes, i.e. melatonin are preserved.

In a specific embodiment the sample material is a bodily fluid. The term "bodily fluid" as used herein refers to whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, liquid stool, fluid harvested from a site of an immune response, fluid harvested from a pooled collection site, bronchial lavage, and urine.

In further embodiments also material such as biopsy material, e.g. from all suitable organs, e.g. the lung, the muscle, brain, liver, skin, pancreas, stomach, etc., a nucleated cell sample, a fluid associated with a mucosal surface or skin may be used. For such a testing, the material is typically homogenized and/or resuspended in a suitable buffer solution.

Additionally, cells may be purified from obtained body tissues and fluids if necessary, and then used as the biological sample. Samples, in particular after initial processing, may be pooled. However, also non-pooled samples may be used.

In further embodiments bodily fluid or sample material as mentioned herein above may be processed by adding chemical or biological reactants. This may be performed in order to stabilize the sample material, to remove sample components, or to avoid interaction in samples. For example, EDTA or heparin may be used to stabilize blood samples, or which prevent coagulation.

In a specific embodiment of the present invention the content of a sample may be submitted to an enrichment step. For instance, a sample may be contacted with ligands specific for the cell membrane or organelles of certain cell types functionalized for example with magnetic particles. The material concentrated by the magnetic particles may subsequently be used for detection and analysis steps as described herein above or below.

It is particularly preferred using salvia, blood, i.e. whole blood, serum, plasma, urine or sweat. In a more preferred embodiment, the sample may be a whole blood or a plasma sample.

A "predefined amount of a compound comprising a derivative of melatonin as mentioned herein above" may be a suitable amount of the derivative whose concentration and in particular whose amount of immunologically active and thus recognizable antigenic structures is known or can be determined. This concentration or amount is preferably adapted to the expected amount of melatonin in a certain sample volume. In specific embodiments, the predefined amount of a compound comprising a derivative of melatonin as mentioned herein above may be adapted to the number or amount of melatonin binding molecules immobilized on a surface. Such an adaptation may, for example, involve the use of the same or essentially the same number of compounds comprising a derivative of melatonin as mentioned herein above, as the number or amount of melatonin binding molecules. In further specific embodiments, the predefined amount may be defined according to an establishment of a functional ratio which is a balance between signal without melatonin and low spike levels. The concentration of binding molecules, e.g. antibodies, or of compounds comprising a derivative of melatonin, may be changed and may be used in order to optimize the adaptation.

The "incubation" and the subsequent "binding" of melatonin and/or the compound comprising a melatonin derivative according to the present invention with a melatonin binding molecule may be performed at any suitable temperature and/or during any suitable period of time. Preferably, the incubation and binding is carried out at a temperature between about 4°C and about 37°C. It is preferred that the incubation is performed at a temperature of more than about 18°C, or more than about 20°C. For example, the incubation may be carried out at a temperature of 18°C, 19°C, 20°C, 21°C, 22°C, 23°C, 24°C, 25°C, 26°C, 27°C, 28°C, 29°C, 30°C, 31°C, 32°C, 33°C, 34°C or 35°C or any value in between the mentioned values. Preferably, the incubation and binding is performed at a temperature of about 18°C to about 25°C, more preferably at 20°C. The incubation and binding may further be performed during a time interval of more or less than 16 hours, preferably during less than 16 hours. It is particularly preferred that the incubation and binding is performed for, e.g. 15 h, 14 h, 13 h, 12 h, 11 h, 10 h, 9 h, 8 h, 7 h, 6 h, 5 h, 4 h, 3 h, 2 h, 1 h, or less than 1 h, or any time period in between the mentioned time periods. In an even more preferred embodiment, the time period for incubation and binding is less than about 1 h, e.g. 50 min, 45 min, 40 min, 35 min, 30 min, 25 min, 20 min, 15 min, 10 min or 5 min or any time period in between these values. Particularly preferred is a time period for incubation and binding of about 10 min.

A "melatonin binding molecule" as mentioned in the context of the above described method and assay may any molecule which is able to specifically bind melatonin and also a melatonin derivative according to the present invention. Such binding molecule may be an immunoglobulin-based protein, or a non-immunoglobulin-based molecule.

A typical and preferred example of an immunoglobulin-based protein is an antibody. The term "antibody" as used herein refers to immunoglobulin molecules and immunologically active portions or fragments of immunoglobulin molecules, i.e. molecules that contain an antigen binding site that immunospecifically binds an antigen, in particular specifically binds melatonin or a melatonin derivative as defined herein above. The immunoglobulin molecules of the invention can be of any type (e.g., IgG, IgE, IgM, IgD, IgA and IgY), class (e. g., IgG1, IgG2, IgG3, IgG4, IgA1 and IgA2) or subclass of immunoglobulin molecule. In a specific embodiment the antibody or fragment thereof as mentioned comprises a human IgM heavy chain constant domain, a human IgGl heavy chain constant domain, a human IgG2 heavy chain constant domain, a human IgG3 heavy chain constant domain, a human IgG4 heavy chain constant domain, or a human IgA heavy chain constant domain. In a further embodiment of the present invention the antibody or fragment, in particular the antibody or fragment thereof comprising a human IgM heavy chain constant domain, a human IgGl heavy chain constant domain, a human IgG2 heavy chain constant domain, a human IgG3 heavy chain constant domain, a human IgG4 heavy chain constant domain, or a human IgA heavy chain constant domain comprises a human Ig kappa light chain constant domain, or a human Ig lambda light chain constant domain. The immunospecific binding refers to the immunospecific detection and binding of an antibody to an antigenic epitope of melatonin and/or a melatonin derivative as defined herein above.

In further embodiments antibodies of the invention include polyclonal, monoclonal, multispecific, human, humanized or chimeric antibodies, whole immunoglobulin molecules, anti-idiotypic (anti-Id) antibodies (including, e. g., anti-Id antibodies to antibodies of the invention), antibody substructures, or modified antibodies.

The term "antibody substructure" as used herein refers to single chain antibodies, Fab fragments, Fab' fragments, fragments produced by a Fab expression library, F(ab')2, Fv, disulfide linked Fv, minibodies, diabodies, scFv, sc(Fv)2, and epitope-binding fragments of any of the above. Preferred are Fab, Fab' and F (ab')2, Fv, single-chain Fvs (scFv), sc(Fv)2, single-chain antibodies, disulfide-linked Fvs (sdFv) and fragments comprising either a VL or VH domain. Also envisaged are VHH (camelid) structures.

The term "Fab fragment" as used herein refers to antibody fragments consisting of the first constant domain of the heavy chain (CH1), the constant domain of the light chain (CL), the variable domain of the heavy chain (VH) and the variable domain of the light chain (VL) of an intact immunoglobulin protein. Typically, Fab fragments are obtained by proteolytic cleavage by papain.

The term "F(ab')2" or "F(ab')2 fragment" as used herein refers to antibody fragments consisting of two first constant domains of the heavy chain (CH1), two constant domains of the light chain (CL), two variable domains of the heavy chain (VH) and two variable domains of the light chain (VL) of an intact immunoglobulin protein, i.e. it comprises two Fab fragments. Additionally, F(ab')2 molecules comprise a S-S linkage in the antibody hinge region which combines the Fab fragments. Typically, F(ab')2 fragments are obtained by proteolytic cleavage by pepsin.

The term "Fab' fragment" as used herein refers to fragments derived from "F(ab')2" molecules, preferably fragments comprising the S-S linkage in the antibody hinge region.

The term "Fv fragments" as used herein refers to antibody fragments consisting of the two variable antibody domains VH and VL (details may be derived from Skerra and Plückthun, 1988, Science, 240: 1038-1041).

The term "single chain Fv fragment (scFv)" as used herein relates to antibody fragments consisting of the two VH and VL domains linked together by a flexible peptide linker (details may be derived from Bird and Walker, 1991, Trends Biotechnol., 9: 132-137).

The term "diabody" as used herein refers to an antibody variant comprising a separated VH-VL and VL-VH fusion, wherein the fused domains are linked together by a flexible peptide linker. The linker may have a length of about 1 to 20 amino acids, preferably of between about 2 and 7 amino acids. Typically, small amino acids like glycine may be used for the linker. They may also be combined with other amino acids (details may be derived from Hudson and Kortt, 1999, J. Immunol. Methods, 231(1-2):177-89).

The term "minibody" as used herein refers to a size reduced antibody, e.g. an antibody comprising solely variable domains or lacking constant domains, or comprising the variable heavy domain (details may be derived from Quiocho, 1993, Nature, 362(6418): 293-294).

Antigen-binding antibody fragments, including single-chain antibodies, may comprise the variable region(s) alone or in combination with the entirety or a portion of the hinge region, CH1, CH2, and/or CH3 domains. Also envisaged are antigen-binding fragments comprising any combination of variable region(s) with a hinge region, CH1, CH2, and CH3 domains. The antibodies may be from any animal origin including birds and mammals. Preferably, the antibodies are murine (e. g., mouse and rat), donkey, monkey, rabbit, goat, guinea pig, camel, horse, chicken, or human.

The antibodies according to the present invention are typically monospecific. In certain specific embodiments, the antibody may also be bispecific, or of a greater multispecificity. Multispecific antibodies may be specific for different epitopes of a melatonin and/or a melatonin derivative as defined herein above, or may be specific for both a melatonin and/or a melatonin derivative as defined herein above as defined herein above, as well as for a heterologous epitope, such as a heterologous polypeptide or solid support material.

The term "modified antibody" as used herein refers to derivatives which are modified, for instance by the covalent attachment of any type of molecule to the antibody such that said covalent attachment does not prevent the antibody from specifically binding to the epitope or from generating an anti-idiotypic response. Typical examples of such modifications are glycosylation, acetylation, biotinylation, PEG-ylation, phosphorylation, amidation, derivatization by known protecting/blocking groups, proteolytic cleavage, linkage to a cellular ligand or other protein, etc. Chemical modifications may be carried out by known techniques, including specific chemical cleavage, acetylation, formylation etc. Additionally, the derivative may contain one or more non-classical amino acids.

Antibodies may be produced according to any suitable method known to the person skilled in the art. Polyclonal antibodies may be produced by immunization of animals with the antigen of choice. For example, melatonin or a melatonin derivative as defined herein above can be administered to various host animals including any eukaryotic, prokaryotic, or phage clone. Monoclonal antibodies of defined specificity may be produced using, for instance, the hybridoma technology developed by Köhler and Milstein (Köhler and Milstein, 1976, Eur. J. Immunol., 6: 511-519). Typically, mice are immunized with melatonin and/or a melatonin derivative as defined herein above. Further details on suitable approaches and techniques can be derived from Huisman et al., 2009, Journal of Neurochemistry, 10.1111/j.1471-4159.2009.06492.x. Once an immune response is detected, e.g., antibodies specific for the antigen are detected in the mouse serum, the mouse spleen is harvested and splenocytes isolated. The splenocytes are then fused by well known techniques to any suitable myeloma cells, for example cells from cell line SP20. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Alternatively, antibodies of the present invention can also be generated using various phage display methods known in the art. In phage display methods, functional antibody domains are displayed on the surface of phage particles, which carry the polynucleotide sequences encoding them. In a particular embodiment, such phage can be utilized to display antigen binding domains expressed from a repertoire or combinatorial antibody library (e. g., human or murine). Phages expressing an antigen binding domain that binds the antigen of interest can be selected or identified with antigen, e. g., using labeled antigen or antigen bound or captured to a solid surface or bead. Phages used in these methods are typically filamentous phages including M13 binding domains expressed from a phage with Fab, Fv or disulfide stabilized Fv antibody domains recombinantly fused to either the phage gene III or gene VIII protein. Examples of phage display methods that can be used to produce antibodies according to the present invention include those disclosed in Brinkman et al., 1995, J. Immunol. Methods 182: 41-50.

In a further embodiment the specifically binding molecule is a non-immunoglobulin molecule. The term "non-immunoglobulin molecule" refers to a group of highly affine molecules which are capable of specifically binding to target molecules such as melatonin and/or a melatonin derivative as defined herein above, but do not comprise immunoglobulin domains or elements. The non-immunoglobulin molecules may offer several distinct mechanisms of binding and preferably have a similar affinity for target structures such as melatonin and/or a melatonin derivative as defined herein above as antibodies.

Examples of non-immunoglobulin molecules which may be used in the context of the present invention include protein structures comprising ankyrin-repeats. Typically, in designed ankyrin-repeat proteins (DARPins) three, four or preferably five repeat ankyrin motifs are present. These may form a stable protein domain with a large potential target interaction surface. Further details may be derived, for example, from Binz et al., 2003, J. Mol. Biol.; 332(2): 489-503.

A further example of a highly affine non-immunoglobulin molecule is an affibody molecule, i.e. a protein based on the Z domain (the immunoglobulin G binding domain) of protein A. In contrast to antibodies, affibody molecules are typically composed of alpha helices and lack disulfide bridges. They may be expressed in soluble and proteolytically stable forms in various host cells. Affibody molecules may further be fused with other proteins. Further details may be derived, for example, from Nord et al., 1997, Nat. Biotechnol.; 15(8): 772-777.

The group of highly affine non-immunoglobulin molecules according to the present invention also comprises adnectins. Adnectins are based on the structure of human fibronectin, in particular its extracellular type III domain, which has a structure similar to antibody variable domains, comprising seven beta sheets forming a barrel and three exposed loops on each side corresponding to the three complementarity determining regions.

Adnectins typically lack binding sites for metal ions and central disulfide bonds. They are approximately 15 times smaller than an IgG type antibody and comparable to the size of a single variable domain of an antibody. Adnectins may be customized in order to generate and/or increase specificity for target molecules by modifying the loops between the second and third beta sheets and between the sixth and seventh sheets. Further details may be derived, for example, from Koide and Koide, 2007, Methods Mol. Biol.; 352: 95-109.

A further preferred example is the antibody mimetic anticalin, which is derived from human lipocalin. Anticalins typically have the property of binding protein antigens, as well as small molecule antigens. They are composed of a barrel structure formed by 8 antiparallel beta sheets, connected by loops and an attached alpha helix. Mutagenesis of amino acids at the binding site may allow for changing of affinity and selectivity of the molecule. Further details may be derived, for example, from Skerra, 2008, FEBS J., 275 (11): 2677-83.

Another preferred example is affilin, i.e. a genetically engineered protein with the ability to selectively bind antigens, which is structurally derived from gamma-B crystallin or from ubiquitin. Affilins are typically constructed by modification of near-surface amino acids of gamma-B crystallin or ubiquitin and isolated by display techniques such as phage display. The molecular mass of crystallin and ubiquitin based affilins is typically about one eighth or one sixteenth of an IgG antibody, respectively. This may lead to heat stability up to 90°C and an improved stability towards acids and bases. Further details may be derived, for example, from Ebersbach et al., 2007 J Mol Biol.; 372(1): 172-185 or from Hey et al., 2005, Trends Biotechnol.; 23(10): 514-522.

The group of highly affine non-immunoglobulin molecules also comprises avimers, i.e. artificial proteins that are able to specifically bind to certain antigens via multiple binding sites. Typically, the individual avimer sequences are derived from A domains of various membrane receptors and have a rigid structure, stabilized by disulfide bonds and calcium. Each A domain can bind to a certain epitope of the target molecule. The combination of domains binding to different epitopes of the same target molecule may increases affinity to this target. Further details may be derived, for example, from Silverman et al., 2005, Nat. Biotechnol.; 23(12): 1556-61.

Further examples include knottins, i.e. small disulfide-rich proteins characterized by a special disulfide through disulfide knot. This knot is typically obtained when one disulfide bridge crosses the macrocycle formed by two other disulfides and the interconnecting backbone (disulfide III-VI goes through disulfides I-IV and II-V). Knottin peptides could be shown to bind with high affinity (about 10 to 30 nmol/L) to integrin receptors. The knottin scaffold may accordingly be used for the design of highly affine molecules which are able to bind detection moieties according to the invention. Further details may be derived, for example, from Kimura et al., 2009, Cancer Res., 69; 2435.

The group of highly affine non-immunoglobulin molecules additionally comprises fynomers, i.e. Fyn SH3-derived proteins. Fyn is a 59-kDa member of the Src family of tyrosine kinases. The Fyn SH3 domain comprises 63 residues, and its amino acid sequence is fully conserved among man, mouse, rat, and monkey. Fynomers are typically composed of two antiparallel beta sheets and contain two flexible loops (RT and n-Src loops) to interact with other proteins or targets. Further details may be derived, for example, from Grabulovski et al., 2007, Journal of Biological Chemistry, 282 (5): 3196-3204.

The group of preferred highly affine non-immunoglobulin molecule also comprises kunitz domain peptides. Kunitz domains are the active domains of Kunitz-type protease inhibitors. They typically have a length of about 50 to 60 amino acids and a molecular weight of 6 kDa. Examples of Kunitz-type protease inhibitors are aprotinin, Alzheimer's amyloid precursor protein (APP), and tissue factor pathway inhibitor (TFPI). Kunitz domains are stable as standalone peptides and are able to recognize specific targets such as protein structure and may accordingly be used for the design of highly affine molecules which are able to bind detection moieties according to the invention. Further details may be derived, for example, from Nixon and Wood, 2006, Curr. Opin. Drug Discov. Devel., 9(2), 261-268.

A melatonin binding molecule as defined herein above is provided in the context of the above mentioned method and assay in an immobilized form. Thus, the binding molecule, e.g. antibody, is immobilized on a surface or support material of a device, chamber or other suitable entity, where binding and measurement reactions are performed. The term "immobilization" or "immobilized" as used herein refers to the association of a melatonin binding molecule to a surface or a support material via molecular interactions which position the melatonin binding molecule at a specific area of the surface or support material and concomitantly impede a detaching of the binding molecule, e.g. during treatment steps, such as washing or rinsing steps etc. Typically, such molecular interactions are based on covalent chemical bonds or covalent chemical interactions between structural elements or functional groups of the surface or support material and the molecule to be immobilized, e.g. corresponding functional groups of the molecule, as known to the person skilled in the art. Also envisaged is an immobilization or attachment of the melatonin binding molecule to a support material by non-covalent interactions. The immobilization may, for example, be carried out by crosslinking the binding molecule by heat or light, i.e. by forming molecular interactions or bonds that link both structural elements together under the influence or driven by the energy provided by an energy source like heat or light, or via a chemical immobilization. Preferred is the drying of a solution comprising the entity to be immobilized, e.g. an antibody or other binding molecule, on the surface. A "chemical immobilization" as mentioned herein may be an interaction between the support material and the binding molecule based on chemical reactions. Such a chemical reaction can be enhanced by either applying heat, e.g. a certain optimal temperature for a chemical reaction. For example, a chemical immobilization may take place between functional groups on a support material and corresponding functional elements on the binding molecules. Such corresponding functional elements in the binding molecules may either be as part of the chemical inventory of a molecule, or be additionally introduced.

An example of such a functional group is an amine group. Typically, the binding molecule to be immobilized, e.g. a nucleic acid or protein, comprises a functional amine group or is chemically modified in order to comprise a functional amine group. For example, the amino group may react with activated groups on the support material, such as a carboxy activated group. Examples include carbonyl, epoxy or similar groups. Means and methods for such a chemical modification are known to the person skilled in the art. The localization of said functional groups within the binding molecule to be immobilized may be used in order to control and shape the binding behavior and/or orientation of the binding molecule. A typical reaction partner for a binding molecule to be immobilized comprises moieties which are capable of binding to such binding molecules such as amine-functionalized nucleic acids.

An "immobilization or attachment of the binding molecule to a support material by non-covalent interactions" may comprise the interaction between the support material and the binding molecule by hydrogen bonding, electrostatic interactions, Van der Waals interactions, and/or hydrophobic packing. Envisaged examples of non-covalent immobilization or attachment of binding molecules to the support material include the use of biotin-streptavidin interactions. For example, a melatonin binding molecule as defined herein, e.g. a DARPin or an antibody, may be coupled to a biotin molecule or be biotinylated. Surfaces or support material may accordingly be provided with streptavidin molecules. Alternatively, similar molecules such as avidin, streptavidin derivatives, (strept)avidin, avidin-related proteins, avidin-like entities such as tamavidin 1 and 2, bradavidin, or NeutrAvidin etc. may be used. These molecules may be coupled in a covalent manner to said support material. Subsequently a non-covalent interaction and attachment, e.g. between the biotinylated molecule and the streptavidin comprising material may be performed, resulting in an immobilization of the melatonin binding molecule to the support material.

Subsequent to the binding of melatonin and/or a compound comprising a melatonin derivative according to the present invention to an immobilized melatonin binding molecule, e.g. an antibody, as defined herein above, washing or rinsing activities may be performed in order to remove non-bound melatonin and/or melatonin derivatives form the surface. Such washing or rinsing steps may be performed according to any suitable protocol, may be carried out one or several times etc.

Upon a removal of non-bound melatonin and/or melatonin derivatives as defined herein above form the surface a measurement of the compound comprising said melatonin derivative bound to said immobilized melatonin binding molecule being may be performed. The measurement may typically be based on the presence of a detectable moiety on or in said compound comprising said melatonin derivative. For example, said compound comprising said melatonin derivative may comprise a label as defined herein above, such as a fluorescent label, a radioactive label or an attached enzyme which is able to perform a detectable enzymatic reaction. In alternative embodiments the measurement may be based on optical detection steps which do not require specific labels, but, for instance, are capable of detecting the presence of particles or carrier structures. Also envisaged is the sensitive detection based on luminol functionalized silver nanoprobe or modified versions thereof. Further details may, for example, be derived from Yu et al., 2014, Analytica Chimica Act, 812, 236-242.

Upon a measurement of the compounds comprising said melatonin derivative bound to said immobilized melatonin binding molecules, it may be deduced, whether all of the binding sites of the immobilized melatonin binding molecules are bound by said compounds comprising said melatonin derivative, or whether a certain percentage of binding sites is not bound by said compounds. The number of not bound binding sites may thus indicate (i) the presence of melatonin in a sample and (ii) allow for a quantification of the amount of melatonin in the sample based on a comparison between the number of binding sites present and the number of sites bound by compounds comprising said melatonin derivative according to the present invention. The quantification is thus, in principle, based on the determination of the inverse proportion of the measured amount of the compounds comprising said melatonin derivative according to the present invention.

In an alternative approach, the present invention provides a method or immunobiological assay for detecting and/or quantifying melatonin in a sample as defined herein above, comprising the steps of: providing a compound comprising a derivative of melatonin according to the present invention in an immobilized form on a surface; or in an immobilizable form; incubating a sample as defined herein above on said surface with a predefined amount of a melatonin binding molecule as defined herein above, thereby allowing for the binding of the melatonin binding molecule to melatonin and/or the immobilized or immobilizable compound comprising said melatonin derivative; measuring the amount of said melatonin binding molecule bound to the immobilized compound comprising said melatonin derivative; and deducing the amount of melatonin in the sample based on the inverse proportion of the measured amount of the melatonin binding molecule.

The alternative approach is thus based on a complementary principle in which instead of the melatonin binding molecule the compound comprising a derivative of melatonin according to the present invention is immobilized or at least immobilizable on a surface. Said immobilization may, for example, be performed according to the above outlined principles. In certain embodiments, the immobilized compound comprising a derivative of melatonin according to the present invention may or may not be coupled to a particle. In further corresponding embodiments, the melatonin binding molecule, e.g. antibody may be coupled to a particle or may not be coupled to a particle as defined herein above.

An example of situation in which no coupling of a derivative of melatonin according to the present invention to a particle is envisaged is provided in the right hand panel of Fig. 3. In this embodiment, a binding molecule as defined herein, e.g. an antibody, may be bound to a particle as defined herein above, e.g. a magnetic particle.

The immobilization may be further based on functionalities of the carrier of the particle being coupled to the derivative of melatonin. For example, in case magnetic particles are used, the immobilization may be performed via magnetic actuation. Such an immobilization may be a temporary or transient immobilization, e.g. carried out during certain steps of the assay. The compound comprising a derivative of melatonin according to the present invention may thus be "immobilizable", but is not necessarily immobilized on a surface during the incubation steps or binding steps. The immobilization may thus, in specific embodiments, be performed after an incubation of a sample with a predefined amount of melatonin binding molecules and with the compound comprising the melatonin derivative according to the present invention.

It is preferred that subsequent to the incubation allowing for the binding of the melatonin binding molecule to melatonin and/or the immobilized or immobilizable compound comprising said melatonin derivative washing or rinsing activities may be performed in order to remove non-bound melatonin and/or melatonin derivatives form the surface. Such washing or rinsing steps may be performed according to any suitable protocol, may be carried out one or several times etc. In case of an immobilizable compound comprising said melatonin derivative, which is freely floating in a sample or mixture comprising a sample, a step of immobilization of the compound to the surface is required before the washing starts, e.g. by magnetic actuation.

Upon a removal of elements which are not bound to said compound comprising said melatonin derivative form the surface, e.g. melatonin and/or non-bound melatonin binding molecules, a measurement of the melatonin binding molecule bound to said compound comprising said melatonin derivative may be performed. The measurement may typically be based on the presence of a detectable moiety on or in said melatonin binding molecule. For example, said melatonin binding molecule may comprise a label as defined herein above, such as a fluorescent label, a radioactive label or an attached enzyme which is able to perform a detectable enzymatic reaction. Preferably, said melatonin binding molecule, e.g. antibody, may comprise an epitope or have an antigenic structure, which can specifically be recognized by a secondary antibody. Such secondary antibody may, in specific embodiments, be labeled as defined herein above. In specific, alternative embodiments the measurement may be based on detection steps which do not require specific labels, but, for instance, are capable of detecting the presence of melatonin binding molecules bound to compounds comprising a derivative of melatonin according to the present invention, e.g. biocore techniques, surface plasmon resonance techniques, or sonic wave techniques, which are based on mass/charge differences

Upon a measurement of the melatonin binding molecules bound to compounds comprising a derivative of melatonin according to the present invention, it may be deduced, whether all of the initially provided melatonin binding molecules are bound by said compounds comprising said melatonin derivative, or whether a certain percentage of melatonin binding molecules is not bound to said compounds. The number of not bound melatonin binding molecules may thus indicate (i) the presence of melatonin in a sample and (ii) allow for a quantification of the amount of melatonin in the sample based on a comparison between the number of melatonin binding molecules initially provided and the number of melatonin binding molecules bound to compounds comprising said melatonin derivative according to the present invention. The quantification is thus, in principle, based on the determination of the inverse proportion of the measured amount of the melatonin binding molecules to said compounds comprising said melatonin derivative according to the present invention. The calculation of the quantitative amounts is typically performed with the help or based upon a dose response curve which is inverse proposotional to the detection spike levels. Such procedure would be known to the skilled person or can be derived from suitable text books such as the ebook Assay Guidance Manual, edited by G. Sitta Sittampalam, Bethesda (MD): Eli Lilly & Company and the National Center for Advancing Translational Sciences; 2004 at http://www.ncbi.nlm.nih.gov/books/NBK53196/, in particular section Immunoassay Methods, Karen L. Cox et al., Eli Lilly & Company, Indianapolis, IN, of 2012, or later updated versions thereof.

In specific embodiments, the methods or assays as defined herein above may be carried with a control or reference sample, e.g. a sample with a known amount of melatonin, or a blank sample which does not comprise melatonin. Such a reference performance, which may be carried out in parallel to a performance of the method/assay with a real sample, may allow for comparison and/or quality-control of obtained measurements, or be used for calibration purposes. Preferably, quality-control samples may comprise buffers with one or more known amounts of melatonin.

Particularly envisaged by the present invention is the specific use of magnetic particles as defined herein above, which can be actuated by applying a magnetic field such that the analytical procedure can be accelerated. It is also envisaged by the present invention that the use of a magnetic field may reduce the background signal due to removal of non-specifically bound particles. Such an approach may, for example, be performed in an optomagnetic. Thus, an assay or method according to the present invention may comprise the one or more steps of magnetically actuating compounds comprising a derivative of melatonin coupled to magnetic particles as defined herein above. Accordingly, the present invention envisages that the method or assay as defined herein above performed in a device allowing magnetic actuation of particles. The actuation typically includes the performance of magnetic forces to the device and/or the particles present in said device.

In one embodiment of the present invention a magnetic force is applied to bring the particles into close proximity with the sensor surface.

In another preferred embodiment of the present invention the detection of bound particles, e.g. magnetic particles, occurs via frustrated total internal reflection (FTIR) or via measurement of scattered light from said bound particles near the surface or via the optical detection of cluster formation. Particularly preferred are sensing devices based on an optical detection of particles, especially magnetic particles as defined herein above. An exemplary device may comprise a light source and a light detection system. The optical methods used for detection typically measure a change in light signal, i.e. a difference in light reflected from the magnetic particles and which can be detected by optical means.

For instance, such methods may include techniques such as the detection of scattered light or detection based on total internal reflection (TIR) or frustrated total internal reflection (FTIR). Preferably, the change in light signal refers to only those magnetic particles being bound by virtue of the binding of the third capture entity to the sensor surface. Details would be known to the person skilled in the art, or can be derived from suitable references, such as Bruls et al., Lab Chip, 2009, 9. 2504-3510.

As used herein the term "total internal reflection" describes a condition present in certain materials when light enters one material from another material with a higher refractive index at an angle of incidence greater than a specific angle. The specific angle at which this occurs depends on the refractive indices of both materials, also referred to as critical angle and can be calculated mathematically (Snell's law, law of refraction). In absence of particles, e.g. magnetic particles, no refraction occurs and the light beam from the light source is totally reflected. If a particle, e.g. magnetic particle, is close to the surface or is in contact with the sensor surface the light rays are said to be frustrated by the particle and reflection at that point is no longer total. The signal, which may be defined as the decrease of the totally internal reflected signal can be calculated.

The signal is more or less linearly dependent on the concentration of particles on the surface (surface density $\tilde{n}$). The signal can be expressed as:

$$S = \beta\,\tilde{n}$$

wherein S is the measured signal change in % and $\beta$ is a conversion factor from surface density to signal change.

In a preferred embodiment of the present invention detection of bound particles, e.g. magnetic particles, occurs via frustrated total internal reflection (FTIR) or via measurement of scattered light from said bound particles near the surface.

The detection may, in a specifically preferred embodiment be carried out in an optomagnetic system, wherein said particles are magnetic particles which are magnetically actuated and optically detected in a stationary sample fluid. A device allowing magnetic actuation of particles may thus preferably be such an optomagentic system.

In a final aspect the present invention relates to a kit of parts for detecting and/or quantifying melatonin, comprising a derivative of melatonin as defined herein above and a melatonin specific antibody as mentioned herein above. Alternatively, a different melatonin binding molecule as defined herein above may be present in the kit, e.g. a non-immunoglobulin molecule as defined herein above. The kit may, in specific embodiments, be provided as a diagnostic kit for diseases associated with increased or decreased levels of melatonin in a sample, e.g. in a sample as defined herein above, in particular, in a sample of saliva, blood, serum, plasma, urine, or sweat.

The ingredients of the kit may, according to the present invention, be comprised in one or more containers or separate entities. They may preferably be formulated as diagnostic or quantification compositions, e.g. they may comprise suitable carriers, or be provided in suitable buffers etc. Examples of accessory ingredients provided in the kit are buffers, sample stabilizing molecules, ions like bivalent cations or monovalent cations, saturation solutions, secondary affinity ligands like, e.g. secondary antibodies, detection dyes, enzymatic substrates and any other suitable compound or liquid necessary for the performance of a detection based on the principle of the present invention, which is known to the person skilled in the art. In specific embodiments, the kit may further comprise reference reagents or control reagents allowing for the performance of calibration or comparison tests.

The kit according to the present invention may optionally also comprise a documentation which indicates the use or employment of the kit and its components. Preferably, instructions comprised in the kit of the present invention may comprise recommended diagnostic options, reference values for quantification and control tests etc. The kit may also comprise an instruction leaflet and/or may provide additional information on the use etc.

The following examples and figures are provided for illustrative purposes. It is thus understood that the example and figures are not to be construed as limiting. The skilled person in the art will clearly be able to envisage further modifications of the principles laid out herein.

EXAMPLES

*Example 1 - Assay format*

In the final assay format, beads coupled with dextran melatonin are dried onto the laminate of the cartridge and after sample addition pulled towards the surface using magnetic force. The coated magnetic particles are captured on the surface of the base part via coated anti-melatonin antibodies (see Figure 3). Presence of melatonin in the sample prevents the binding of the coated beads, which results in decreased blackness of the coated spot and thus a lower signal read out.

*Example 2 - Conjugation of melatonin to amino dextran*

Melatonin was coupled to amino-dextran using EDC. In brief, 19 $\mu$l EDC (10mg/ml in water) was mixed with 5$\mu$l Melatonin-COOH (20mg/ml in DMSO), and 130 $\mu$l Aminodextran40 (10 mg/ml in 50 mM MES pH=6.2) and incubated for 1 hour rolling on a Roller bench. The uncoupled melatonin was removed by PD10 desalting against MES. The efficiency of the coupling was determined by spectrophotometric analysis and assuming 100% recovery of the dextran after desalting.

*Example 3 - Fluidic part*

Dextran-Mel Bead coating

Beads were transferred to a reaction vial and washed three times with 50mM MES pH=6.2 using a magnetic particle concentrator. The magnetic beads were collected and suspended to a final concentration of 20mg/ml. The carboxylated beads were activated for 30 minutes with 12.5mM EDC and 21mM NHS in 50mM MES pH6.2. After activation the beads were washed with 50mM MES pH6.2 and mixed with an equal volume of the dextran-melatonin conjugates (desalted to 50mM MES pH=6.2) to final concentration of 1 or 10$\mu$g/ml dextran (Melatonin-1-propionic acid or GUS). After 30 minutes incubation on a roller bench, the reaction was stopped by addition of 2% blockmaster CE510 (JSR cooperation) to a final concentration of 0.4%. The beads were incubated overnight on a roller bench. The beads were inspected on presence of aggregated (clusters of) beads. Uncoupled dextran conjugate was removed by washing, three times with 10mM HEPES pH=7.4 / 250mM KCl/ 250mM KBr / 20% Sucrose / 0.05% pluronic-F127/ 0.09% $NaN_3$ and stored at 4°C in this buffer at a concentration of 5.33 g/l until needed.

Preparation of the laminates

Beads were transferred to a reaction vial and collected using a Magnetic particle concentrator. Next, the collected beads were resuspended in dry buffer (10mM HEPES pH=7.4/10%FCS/50mM KCl/50mM KBr/20% Sucrose/0.05% pluronic-F127/0.09% $NaN_3$) and 150nl was dosed using the Nanodrop™ (Innovadyne™ - IDEX® Health & Science) on each laminate. The dosed bead solution was dried for 30minutes at 37°C and stored in a Totech cabinet (Totech Europe B.V, Netherlands) until needed.

*Example 4 - Base part and melatonin standard series*

Printing of antibodies

The antibodies 1-76-4 (Salimetrics, USA) were diluted to 0.5 or 1 $\mu$g/ml (Melatonin-1-propionic acid or GUS) in 50mM BTP pH=6.8 supplemented with 80$\mu$g/ml BSA. The antibodies were printed using the sciFLEXARRAYER (SCIENION AG), printing 2 spots of ≈2nl in each chamber. After printing the parts were stored overnight at 37°C and the hydrophilized with filling buffer (10mM Phosphate pH7.4, 2% Sucrose, 0.1% Goat IgG, 0.1% Saponin, 0.09% $NaN_3$).

Melatonin standard series

Melatonin (Sigma) dissolved in 33% EtOH was used to prepare standard series in plasma ranging from 0 to 1000pg/ml. The standard series were stored in 100$\mu$l aliquots at - 20°C.

*Example 5 - Comparison of the assay formats*

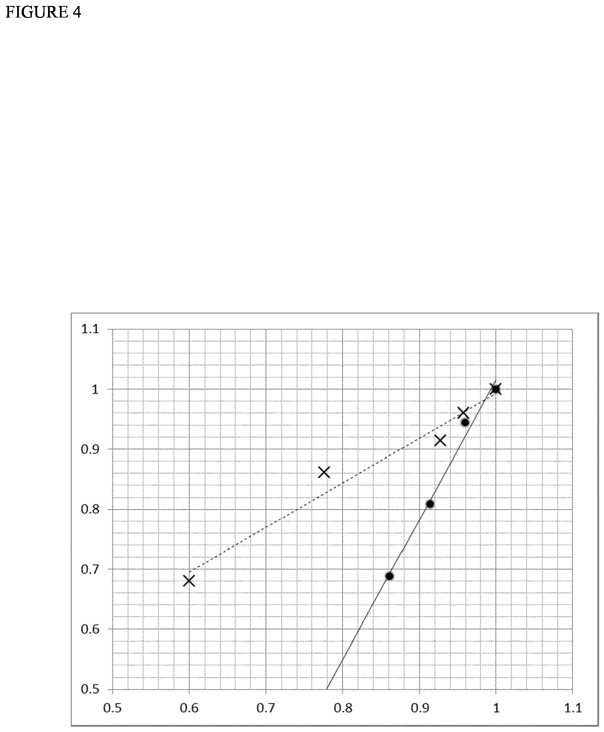

A complete dose response curve was made with the reference conjugate and GUS conjugate an assay temperature of 20°Cand 30°C (for the GUS conjugate) and melatonin dilution series in plasma. We found similar inhibition with both constructs when we measured the GUS conjugate at 30°C and the reference conjugate at 20°C, while improved inhibition was observed when we tested both conjugates at 20°C (see Figure 4).

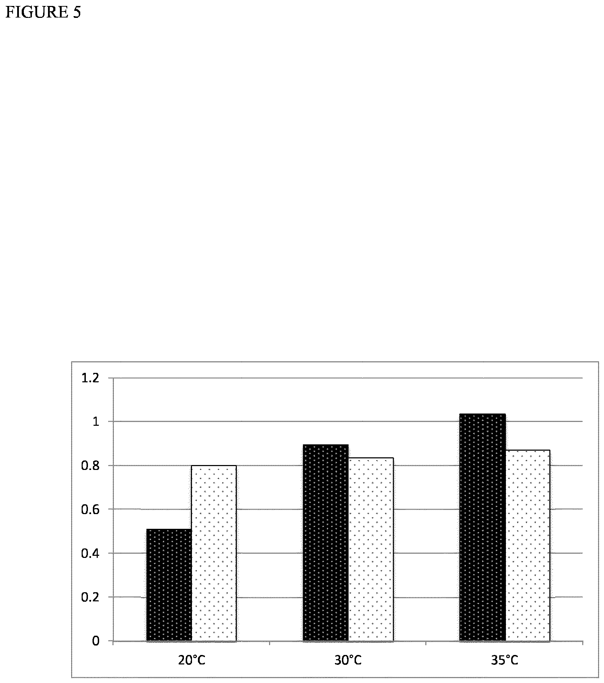

The temperature dependence of both conjugates was investigated with both conjugates at 20pg/ml. For both conjugates we observe a temperature dependency, but clearly less compared for the GUS conjugates (see Figure 5).

**Claims**

1. Use of a derivative of melatonin in an immunobio logical assay, wherein said derivative is a conjugate at position 3 of melatonin's indole ring and wherein said conjugate comprises a linker of at least 2 carbon atoms, with the proviso that the conjugate does not comprise a polypeptide or protein antigen, wherein said derivative is coupled to a carrier, wherein said carrier is further coupled to a particle, or is coating a surface.

2. The use of claim 1, wherein said derivative is or comprises 3-(2-ethylamidoglutaric acid)-5-methoxyindole (GUS).

3. The use of claim 1, wherein said carrier is dextran.

4. The use of any one of claims 1 to 3, wherein said compound comprising a melatonin derivative and a carrier is labeled.

5. The use of claim 1, wherein said assay is an immunobio logical melatonin assay with a sensitivity of <10 pg melatonin/ml, preferably an immunobio logical melatonin quantification assay.

6. The use of claims 1, wherein said assay is performed at a temperature of more than about 20°C, preferably at a temperature of about 20°C to 25°C and/or wherein said assay is performed for less than 16 h, preferably for about 0.5 h.

7. Method or immunobiological assay for detecting and/or quantifying melatonin in a sample, comprising the steps of:

   (a) incubating a sample with a predefined amount of a compound comprising a derivative of melatonin as defined in any one of claims 1 to 6;
   (b) binding of melatonin and/or the compound comprising said melatonin derivative with a melatonin binding molecule, wherein said melatonin binding molecule is immobilized on a surface;
   (c) measuring the amount of the compound comprising said melatonin derivative bound to said melatonin binding molecule; and
   (d) detecting the presence of melatonin in the sample and/or deducing the amount of melatonin in the sample based on the inverse proportion of the measured amount of the compound comprising said melatonin derivative.

8. Method or immunobiological assay for detecting and/or quantifying melatonin in a sample, comprising the steps of:

   (a) providing a compound comprising a derivative of melatonin as defined in any one of claims 1 to 6 in an immobilized form on a surface, or in an immobilizable form;
   (b) incubating a sample on said surface with a predefined amount of a melatonin binding molecule thereby allowing for the binding of the melatonin binding molecule to melatonin and/or the immobilized or immobilizable compound comprising said melatonin derivative;
   (c) measuring the amount of said melatonin binding molecule bound to the immobilized compound comprising said melatonin derivative; and
   (d) deducing the amount of melatonin in the sample based on the inverse proportion of the measured amount of the melatonin binding molecule.

9. The method or assay of claim 7 or 8, wherein said binding molecule is a melatonin specific antibody.

10. The method or assay of claim 7 or 8, wherein said method or assay has a sensitivity of <10 pg melatonin/ml, performed at a temperature of more than about 20°C, preferably at a temperature of about 20°C to 25°C, and/or performed for less than 16 h, preferably for about 0.5 h.

11. The method or assay of claim 7 or 8, wherein said method or assay is performed in a device allowing magnetic actuation of particles.

12. The method or assay of claim 7 or 8, wherein said sample is a saliva, blood, serum, plasma, urine, or sweat sample.

13. Kit of parts for detecting and/or quantifying melatonin, comprising a derivative of melatonin as defined in any one of claims 1 to 6 and a melatonin specific antibody.

**Patentansprüche**

1. Verwendung eines Melatonin-Derivats in einem Immunassay, wobei das genannte Derivat ein Konjugat an Position 3 des Indolrings von Melatonin ist und wobei das genannte Konjugat einen Linker von mindestens 2 Kohlenstoffatomen umfasst, mit der Maßgabe, dass das Konjugat kein Polypeptid oder Protein-Antigen umfasst, wobei das genannte Derivat an einen Träger gekoppelt ist, wobei der genannte Träger weiterhin an einen Partikel gekoppelt ist oder eine Beschichtung einer Oberfläche ist.

2. Verwendung von Anspruch 1, wobei das genannte Derivat 3-(2-Ethylamidoglutarsäure)-5-methoxyindol (GUS) ist oder umfasst.

3. Verwendung von Anspruch 1, wobei der genannte Träger Dextran ist.

4. Verwendung von einem der Ansprüche 1 bis 3, wobei die genannte Verbindung ein Melatonin-Derivat umfasst und ein Träger gelabelt ist.

5. Verwendung von Anspruch 1, wobei der genannte Assay ein Melatonin-Immunassay mit einer Empfindlichkeit von < 10 pg Melatonin/ml ist, vorzugsweise ein Immunassay zur Quantifizierung von Melatonin.

6. Verwendung von Anspruch 1, wobei der genannte Assay bei einer Temperatur von mehr als ca. 20 °C, vorzugsweise bei einer Temperatur von ca. 20 °C bis 25 °C, durchgeführt wird und/oder wobei der genannte Assay für weniger als 16 Stunden, vorzugsweise für ca. 0,5 Stunden, durchgeführt wird.

7. Verfahren oder Immunassay zum Nachweisen und/oder Quantifizieren von Melatonin in einer Probe, die folgenden Schritte umfassend:

(a) Inkubieren einer Probe mit einer vorgegebenen Menge einer Verbindung umfassend ein Melatonin-Derivat nach einem der Ansprüche 1 bis 6;
(b) Binden von Melatonin und/oder der das Melatonin-Derivat umfassenden Verbindung mit einem melatonin-bindenden Molekül, wobei das genannte melatonin-bindende Molekül auf einem Untergrund immobilisiert wird;
(c) Messen der Menge der Verbindung, die das genannte Melatonin-Derivat gebunden an das genannte melatonin-bindende Molekül umfasst; und
(d) Nachweisen der Anwesenheit von Melatonin in der Probe und/oder Ableiten der Menge an Melatonin in der Probe basierend auf dem umgekehrten Verhältnis der gemessenen Menge der das genannte Melatonin-Derivat enthaltenden Verbindung.

8. Verfahren oder Immunassay zum Nachweisen und/oder Quantifizieren von Melatonin in einer Probe, umfassend die folgenden Schritte:

(a) Bereitstellen einer Verbindung umfassend ein Melatonin-Derivat nach einem der Ansprüche 1 bis 6 in einer immobilisierten Form auf einem Untergrund oder in einer immobilisierbaren Form;
(b) Inkubieren einer Probe auf dem genannten Untergrund mit einer vorgegebenen Menge eines melatonin-bindenden Moleküls, wodurch das Binden des melatonin-bindenden Moleküls an das Melatonin und/oder die immobilisierte oder immobilisierbare Verbindung umfassend das genannte Melatonin-Derivat ermöglicht wird;
(c) Messen der Menge des genannten melatonin-bindenden Moleküls, das an die immobilisierte Verbindung umfassend das genannte Melatonin-Derivat gebunden ist; und
(d) Ableiten der Menge an Melatonin in der Probe basierend auf dem umgekehrten Verhältnis der gemessenen Menge des melatonin-bindenden Moleküls.

9. Verfahren oder Assay nach Anspruch 7 oder 8, wobei das genannte bindende Molekül ein melatoninspezifischer Antikörper ist.

10. Verfahren oder Assay nach Anspruch 7 oder 8, wobei das genannte Verfahren oder der Assay eine Empfindlichkeit von < 10 pg Melatonin/ml hat, bei einer Temperatur von mehr als ca. 20 °C, vorzugsweise bei einer Temperatur von ca. 20 °C bis 25 °C durchgeführt wird, und/oder für weniger als 16 Stunden, vorzugsweise für ca. 0,5 Stunden, durchgeführt wird.

11. Verfahren oder Assay nach Anspruch 7 oder 8, wobei das genannte Verfahren oder der Assay in einer Vorrichtung durchgeführt wird, die eine magnetische Ingangsetzung von Partikeln ermöglicht.

12. Verfahren oder Assay nach Anspruch 7 oder 8, wobei die genannte Probe eine Speichel-, Blut-, Serum-, Plasma-, Urin- oder Schweißprobe ist.

13. Satz von Teilen zum Nachweisen und/oder Quantifizieren von Melatonin, umfassend ein Melatonin-Derivat nach einem der Ansprüche 1 bis 6 und einen melatoninspezifischen Antikörper.

## Revendications

1. Utilisation d'un dérivé de mélatonine dans un dosage immunobiologique, dans laquelle ledit dérivé est un conjugué en position 3 du cycle indole de la mélatonine et dans laquelle ledit conjugué comprend un liant d'au moins 2 atomes de carbone, à condition que le conjugué ne comprenne pas d'antigène polypeptidique ou protéique, dans laquelle ledit dérivé est couplé à un vecteur, dans laquelle ledit vecteur est en outre couplé à une particule, ou recouvre une surface.

2. Utilisation selon la revendication 1, dans laquelle ledit dérivé est ou comprend du 3-(2-acide éthylamidoglutarique)-5méthoxyindole (GUS).

**3.** Utilisation selon la revendication 1, dans laquelle ledit vecteur est du dextrane.

**4.** Utilisation selon l'une quelconque des revendications 1 à 3, dans laquelle ledit composé comprenant un dérivé de mélatonine et un vecteur est marqué.

**5.** Utilisation selon la revendication 1, dans laquelle ledit dosage est un dosage de mélatonine immunobiologique avec une sensibilité de <10 pg de mélatonine/ml, de préférence un dosage quantitatif de mélatonine immunobiologique.

**6.** Utilisation selon la revendication 1, dans laquelle ledit dosage est réalisé à une température supérieure à environ 20 °C, de préférence à une température d'environ 20 °C à 25 °C et/ou dans laquelle ledit dosage est réalisé pendant moins de 16 heures, de préférence pendant environ 0,5 heure.

**7.** Procédé ou dosage immunologique pour détecter et/ou quantifier la mélatonine dans un échantillon, comprenant les étapes suivantes :

(a) l'incubation d'un échantillon avec une quantité prédéfinie d'un composé comprenant un dérivé de mélatonine selon l'une quelconque des revendications 1 à 6 ;
(b) la liaison de la mélatonine et/ou du composé comprenant ledit dérivé de mélatonine avec une molécule de liaison à la mélatonine, dans lequel ladite molécule de liaison à la mélatonine est immobilisée sur une surface ;
(c) la mesure de la quantité du composé comprenant ledit dérivé de mélatonine lié à ladite molécule de liaison à la mélatonine ; et
(d) la détection de la présence de mélatonine dans l'échantillon et/ou la déduction de la quantité de mélatonine dans l'échantillon sur la base de la proportion inverse de la quantité mesurée du composé comprenant ledit dérivé de mélatonine.

**8.** Procédé ou dosage immunologique pour détecter et/ou quantifier la mélatonine dans un échantillon, comprenant les étapes suivantes :

(a) la fourniture d'un composé comprenant un dérivé de mélatonine selon l'une quelconque des revendications 1 à 6 sous forme immobilisée sur une surface, ou sous forme pouvant être immobilisée ;
(b) l'incubation d'un échantillon sur ladite surface avec une quantité prédéfinie d'une molécule de liaison à la mélatonine permettant ainsi la liaison de la molécule de liaison à la mélatonine à la mélatonine et/ou au composé immobilisé ou pouvant être immobilisé comprenant ledit dérivé de mélatonine ;
(c) la mesure de la quantité de ladite molécule de liaison à la mélatonine liée au composé immobilisé comprenant ledit dérivé de mélatonine ; et
(d) la déduction de la quantité de mélatonine dans l'échantillon sur la base de la proportion inverse de la quantité mesurée de la molécule de liaison à la mélatonine.

**9.** Procédé ou dosage selon la revendication 7 ou 8, dans lequel ladite molécule de liaison est un anticorps spécifique à la mélatonine.

**10.** Procédé ou dosage selon la revendication 7 ou 8, dans lequel ledit procédé ou dosage présente une sensibilité de <10 pg de mélatonine/ml, réalisé à une température supérieure à environ 20 °C, de préférence à une température d'environ 20 °C à 25 °C, et/ou réalisé pendant moins de 16 heures, de préférence pendant environ 0,5 heure.

**11.** Procédé ou dosage selon la revendication 7 ou 8, dans lequel ledit procédé ou dosage est réalisé dans un dispositif permettant l'activation magnétique de particules.

**12.** Procédé ou dosage selon la revendication 7 ou 8, dans lequel ledit échantillon est un échantillon de salive, de sang, de sérum, de plasma, d'urine, ou de transpiration.

**13.** Kit ou parties pour détecter et/ou quantifier la mélatonine, comprenant un dérivé de mélatonine selon l'une quelconque des revendications 1 à 6 et un anticorps spécifique à la mélatonine.

FIGURE 1

FIGURE 2

FIGURE 3

Anti-melatonin antibodies

Melatonin

coated bead

GUS/melatonin derivative coupled to Dextran

FIGURE 4

FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VOULTSIOS et al.** *Journal of Biological Rhythms,* 1997, vol. 12, 457-466
- **RÖMSING et al.** *Scand. J. Clin. Lab. Invest,* 2006, vol. 66, 181-190
- **GROTA et al.** *Can J Biochem Cell Biol,* 1983, vol. 61, 1096-1101
- **CHEGINI et al.** *Clin. Chem,* 1995, vol. 41, 381-386
- **DI et al.** *Clin Chem,* 1998, vol. 44, 304-310
- **RATNER, B.D. ; HOFFMAN, A.S. ; SCHOEN, F.J. ; LEMONS, J.E.** Biomaterials Science. Elsevier, 2004
- Assay Guidance Manual. Eli Lilly & Company and the National Center for Advancing Translational Sciences, 2004
- **KAREN L. COX et al.** particular section Immunoassay Methods. Eli Lilly & Company, 2012
- **SKERRA ; PLÜCKTHUN.** *Science,* 1988, vol. 240, 1038-1041
- **BIRD ; WALKER.** *Trends Biotechnol.,* 1991, vol. 9, 132-137
- **HUDSON ; KORTT.** *J. Immunol. Methods,* 1999, vol. 231 (1-2), 177-89
- **QUIOCHO.** *Nature,* 1993, vol. 362 (6418), 293-294
- **KÖHLER ; MILSTEIN.** *Eur. J. Immunol.,* 1976, vol. 6, 511-519
- **HUISMAN et al.** *Journal of Neurochemistry,* 2009, vol. 10, 1111

- **BRINKMAN et al.** *J. Immunol. Methods,* 1995, vol. 182, 41-50
- **BINZ et al.** *J. Mol. Biol.,* 2003, vol. 332 (2), 489-503
- **NORD et al.** *Nat. Biotechnol.,* 1997, vol. 15 (8), 772-777
- **KOIDE ; KOIDE.** *Methods Mol. Biol.,* 2007, vol. 352, 95-109
- **SKERRA.** *FEBS J.,* 2008, vol. 275 (11), 2677-83
- **EBERSBACH et al.** *J Mol Biol.,* 2007, vol. 372 (1), 172-185
- **HEY et al.** *Trends Biotechnol.,* 2005, vol. 23 (10), 514-522
- **SILVERMAN et al.** *Nat. Biotechnol.,* 2005, vol. 23 (12), 1556-61
- **KIMURA et al.** *Cancer Res.,* 2009, vol. 69, 2435
- **GRABULOVSKI et al.** *Journal of Biological Chemistry,* 2007, vol. 282 (5), 3196-3204
- **NIXON ; WOOD.** *Curr. Opin. Drug Discov. Devel.,* 2006, vol. 9 (2), 261-268
- **YU et al.** *Analytica Chimica Act,* 2014, vol. 812, 236-242
- **KAREN L. COX et al.** Immunoassay Methods. Eli Lilly & Company, 2012
- **BRULS et al.** *Lab Chip,* 2009, vol. 9, 2504-3510